# EUROPEAN PATENT SPECIFICATION

(11) **EP 3 087 397 B1**
(45) Date of publication and mention of the grant of the patent: **24.10.2018**
(21) Application number: 14874350.3
(22) Date of filing: 24.11.2014
(51) Int. Cl.: G01N 33/68

(54) **METHOD OF DISTINGUISHING BETWEEN DIFFERENT NEURODEGENERATIVE DISEASES**
VERFAHREN ZUR UNTERSCHEIDUNG ZWISCHEN VERSCHIEDENEN NEURODEGENERATIVEN ERKRANKUNGEN
PROCÉDÉ DE DISTINCTION DE DIFFÉRENTES MALADIES NEURODÉGÉNÉRATIVES

(30) Priority: 23.12.2013 US 201361920041 P
(43) Date of publication of application: 02.11.2016
(73) Proprietor: Arizona Board of Regents on behalf of Arizona State University, Scottsdale, Arizona 85257-3538 (US)
(72) Inventor: SIERKS, Michael, Ft. McDowell, Arizona 85264 (US); WILLIAMS, Stephanie, Phoenix, Arizona 85043 (US)
(74) Representative: Comoglio, Elena
(86) International application number: PCT/US2014/067169
(87) International publication number: WO 2015/099931

(56) References cited:
- WO-A1-2007/022015
- WO-A1-2012/058334
- WO-A2-2006/070290
- ZAMEER A ET AL: "Anti-oligomeric Abeta Single-chain Variable Domain Antibody Blocks Abeta-induced Toxicity Against Human Neuroblastoma Cells", JOURNAL OF MOLECULAR BIOLOGY, ACADEMIC PRESS, UNITED KINGDOM, vol. 384, no. 4, 26 December 2008 (2008-12-26), pages 917-928, XP025744854, ISSN: 0022-2836, DOI: 10.1016/J.JMB.2008.09.068 [retrieved on 2008-12-03]
- SRINATH KASTURIRANGAN ET AL: "Nanobody specific for oligomeric beta-amyloid stabilizes nontoxic form", NEUROBIOLOGY OF AGING, vol. 33, no. 7, 1 July 2012 (2012-07-01), pages 1320-1328, XP055346057, US ISSN: 0197-4580, DOI: 10.1016/j.neurobiolaging.2010.09.020
- SRINATH KASTURIRANGAN ET AL: "Isolation and characterization of antibody fragments selective for specific protein morphologies from nanogram antigen samples", BIOTECHNOLOGY PROGRESS., vol. 29, no. 2, 1 March 2013 (2013-03-01), pages 463-471, XP055360716, US ISSN: 8756-7938, DOI: 10.1002/btpr.1698
- WILLIAMS S M ET AL: "Detection of oligomeric a-synuclein using a novel sandwich ELISA as a potential early diagnosis of Parkinson's Disease", SOCIETY FOR NEUROSCIENCE ABSTRACT VIEWER AND ITINERARY PLANNER, vol. 43, 2013, XP9193964, & 43RD ANNUAL MEETING OF THE SOCIETY-FOR-NEUROSCIENCE; SAN DIEGO, CA, USA; NOVEMBER 09 -13, 2013
- WILLIAMS STEPHANIE ET AL: "A sensitive phage-based capture ELISA for sub-femtomolar detection of protein variants directly from biological samples.", BIOTECHNOLOGY PROGRESS, vol. 31, no. 1, January 2015 (2015-01), pages 289-298, XP09193965, ISSN: 1520-6033
- STEPHANIE M. WILLIAMS ET AL: "Oligomeric [alpha]-synuclein and [beta]-amyloid variants as potential biomarkers for Parkinson's and Alzheimer's diseases", EUROPEAN JOURNAL OF NEUROSCIENCE., vol. 43, no. 1, 15 October 2015 (2015-10-15), pages 3-16, XP055360682, GB ISSN: 0953-816X, DOI: 10.1111/ejn.13056
- SIERKS, MR ET AL.: 'CSF Levels Of Oligomeric Alpha-Synuclein And Beta-Amyloid As Biomarkers For Neurodegenerative Disease.' INTEGR BIOL vol. 3, no. 12, December 2011, CAMB, pages 1188 - 1196, XP055078211

## Description

### RELATED APPLICATION

This application claims the benefit under 35 U.S.C. § 119(e) of U.S. Provisional Application No. 61/920,041, filed December 23, 2013.

### BACKGROUND

Any disorder caused by the gradual and progressive loss of neural tissue in the central nervous system can be classified as a neurodegenerative disease. Neurodegenerative diseases are specifically associated with the atrophy of the affected central or peripheral nervous system structures. The presence of protein aggregates, called inclusion bodies or Lewy bodies, caused by the misfolding and subsequent aggregation of abnormal proteins is a pathogenic hallmark of neurodegenerative diseases. Alzheimer's disease (AD), the most prevalent neurodegenerative disease which affects more than 5 million United States residents, is caused by the accumulation of misfolded beta-amyloid (Aβ) and tau proteins in the brain. Similarly, aggregated alpha-synuclein (a-syn) is a major component of the Lewy bodies associated with Parkinson's disease (PD). AD, PD, Huntington's disease, amyotrophic lateral sclerosis (ALS), and prion diseases each possess common cellular and molecular mechanisms including abnormal protein aggregation and the formation of inclusion bodies. An emerging theory states that the Lewy bodies and protein masses collectively signify an end state of the pathology, where the pathogenesis occurs prior to the formation of these aggregates (Ross CA, Poirier MA. Protein aggregation and neurodegenerative disease. Nat Med. 2004; 10 Suppl: S10-7; Sierks MR, Chatterjee G, McGraw C, et al. CSF levels of oligomeric alpha-synuclein and beta-amyloid as biomarkers for neurodegenerative disease. Integr Biol. 2011; 3(12): 1188-96; Kasturirangan S, Li L, Emadi S, et al. Nanobody specific for oligomeric beta-amyloid stabilizes nontoxic form. Neurobiol Aging. 2012; 33(7): 1320-8).

Notable advances in the understanding of neurodegenerative diseases have been established over the past 25 years; however, applicable treatments and prevention measures have yet to be established. Currently, 5 million Americans suffer from Alzheimer's disease, 1 million from Parkinson's, 30,000 from ALS, and 30,000 from Huntington's disease. Without the development of disease-modifying therapies, more than 12 million Americans will suffer from neurodegenerative diseases by 2040. There exists a need to develop a method of earlier disease detection, specifically the presymptomatic detection of neuronal dysfunction for neurodegenerative diseases. In this effort, sensitive detection methods are required to assess disease progression.

There are currently no reliable physical diagnostic tests to distinguish between different pre-symptomatic neurodegenerative diseases. Thus, methods are still needed to aid in the diagnosis of these diseases.

### SUMMARY

The present invention provides a method of determining a quantity of a target oligomer in a biological fluid to determine an increased likelihood of having or developing a neurodegenerative disease in a patient, comprising; (a) obtaining the biological fluid; (b) contacting the sample to a solid support, wherein the solid support is coated with a capture agent specific for the target oligomer and is blocked with a blocking agent to form a bound sample; (c) incubating the bound sample; (d) contacting the bound sample with labeled phage specific for the target oligomer to form a labeled bound sample; (e) contacting the labeled bound sample with labeled detection agent to form a visualizable sample; and (f) comparing the quantity of the visualizable sample and to a control to determine the quantity of the target oligomer.

In certain embodiments, the present invention provides a method of distinguishing between different neurodegenerative diseases in a patient comprising: (a) obtaining a biological fluid from the patient; (b) contacting the sample to a solid support, wherein the solid support comprises two or more locations, wherein each location is coated with a different capture agent specific for the target oligomer, and wherein the solid support is blocked with a blocking agent to form a bound sample; (c) incubating the bound sample; (d) contacting the bound sample with two or more biotinylated phages, wherein each biotinylated phage is specific for a single target oligomer to form labeled bound samples; (e) contacting the labeled bound samples with labeled detection agent to form a visualizable samples; and (f) comparing the quantity of the visualizable samples to quantities of controls to determine the quantity of each target oligomer; wherein an increase in toxic oligomeric Aβ is indicative of the patient having early AD, wherein an increase in oligomeric Aβ and tau aggregates is indicative of later stage AD, wherein an increase in oligomeric tau but not oligomeric Aβ is indicative a tauopathy; wherein an increase in oligomeric a-syn is indicative of Parkinson's Disease; and wherein an increase oligomeric Aβ and a-syn is indicative of Dementia with Lewy Bodies (DLB).

If levels of toxic Aβ aggregates are much higher than levels of toxic a-syn and tau aggregates, then neurodegeneration is likely precipitated by Aβ and not a-syn or tau, consistent with AD; if levels of toxic tau aggregates are much higher than Aβ or a-syn aggregates, than neurodegeneration is driven by tau aggregation and not Aβ or a-syn, consistent with tauopathies and perhaps later stage AD; and if levels of toxic a-syn aggregates predominate, then neurodegeneration is driven by a-syn and not Aβ or tau consistent with synucleinopathies such as PD.

### BRIEF DESCRIPTION OF THE DRAWINGS

**Figures 1A and 1B****.** Different Primary Capture scFvs Binding at Varying Temperatures and Lengths of Incubation. Fig. 1A Concentration of scFv 1 Bound to ELISA Wells. Fig. 1B Concentration of scFv 2 Bound to ELISA Wells.
**Figure 2****.** Different Primary Capture scFvs Binding after 2 Hours at 37°C Incubation.
**Figures 3A and 3B****.** Comparison of Amine Biotinylated Phage and scFv and Different Carboxyl Biotinylated Phages. Fig. 3A Titration of Amine Biotinylated Phage and scFv. Fig. 3B Varying Biotinylation Conditions.
**Figures 4A and 4B****.** Ascertain Effective Blocking Solution. Fig. 4A Varying Blocking Solution Concentrations in Indirect ELISA. Fig. 4B Varying Blocking Solutions in Indirect ELISA.
**Figures 5A and 5B****.** Determination of the Optimum Phage and Phage Concentration. Fig. 5A Varying Phage Concentration with 2% Milk as Blocking Solution. Fig. 5B Different Biotinylated Phages with Purified Antigen in Indirect ELISA.
**Figure 6****.** Titration of Aggregated Protein Targets Using D10 40 mmol Carboxyl Biotinylated Phage.
**Figure 7****.** Selection of Avidin-HRP Concentration.
**Figure 8****.** Phage Capture ELISA using scFv1 and Human Brain Samples with different Neurodegenerative Diseases.
**Figure 9****.** Selection of Sample Incubation Conditions. Comparison of 2 Hours Versus Overnight Sample Incubations with scFv 1 in Capture ELISA.
**Figure 10****.** Indirect ELISA Using SuperSignal ELISA Pico Chemiluminescent Kit. Aggregated Proteins with Pico Chemiluminescent Kit.
**Figure 11****.** Capture ELISA Using SuperSignal ELISA Pico Chemiluminescent Kit and Mice Brain Samples.
**Figure 12****.** Optimizing Biotinylated Phage and Avidin-HRP Concentration with 0.1mg/ml of Human Brain Samples and Femto kit
**Figures 13A-13C****.** Comparison of PelB Phage to SRP Phage. Fig. 13A scFv 1 with Mice Samples and the Different Phages. Fig. 13B Comparison of Background Between SRP Phage and PelB Phage. Fig. 13C Raw Values Using Both Phages.
**Figure 14****.** SRP-Phage Capture ELISA using scFv1 and Human Brain Samples with different Neurodegenerative Diseases and Femto Chemiluminescent Kit.
**Figure 15****.** SRP-Phage vs. PelB-Phage in Mouse Brain Sample Titration.
**Figure 16****.** Normalized Relative Concentrations of Human Brain Samples with scFv 1 in SRP-Phage Capture ELISA.
**Figure 17****.** Schematic of AFM biopanning protocol.
**Figure 18****.** AFM images of phage clone (F9T) binding to trimeric but not monomeric tau. Monomeric (A) and trimeric tau (B) were deposited on mica and F9T phage added. Phage can clearly be seen binding many of the trimeric tau targets (B) but not monomeric tau (A). Scale bar is 1 µm.
**Figure 19****.** Anti-oligomeric tau nanobodies (F9T, D11C and H2A) react with brain tissue from 3xTG-AD mice. Two different samples from each age group were analyzed in triplicate. High levels of oligomeric tau are present at 5 months, peak at 9 months and drop by 13 months.
**Figure 20****.** AFM image of phage binding to TDP-43 aggregates derived from diseased brain tissue. Preliminary panning results showing isolation of phage selective for aggregated TDP-43. No phage were observed in a sample of TDP-43 derived from healthy tissue (not shown). Phage binding disease specific TDP-43 are observed as long filaments (see lower right corner).
**Figure 21****.** Schematic of nanobody (light blue) displayed on surface of phage particle (dark blue). Multiple copies of pVIII coat protein enable over 1000-fold enhancement of nanobody signal.
**Figures 22A-22C****.** Oligomeric Aβ levels in human brain tissue from cognitively normal (ND), AD and PD samples. A) Reactivity with A4 nanobody. B) Reactivity with C6T nanobody. C) Combined oligomeric Aβ levels (A4+C6T). Green bar: ND samples with no plaques, Light pink: ND with moderate plaques. Maroon: AD with moderate plaques. Dark purple: AD with severe plaques. Orange: PD samples. The highest oligomeric Aβ levels are observed in AD samples with moderate plaques, then in AD samples with severe plaques.
**Figure 23****.** Oligomeric a-syn levels in human brain tissue from cognitively normal (ND), AD and PD samples probed with 10H nanobody. Orange bar: PD samples, Maroon: AD samples, Green bar: ND samples High oligomeric a-syn levels were present in PD samples but not AD or ND samples.
**Figure 24****.** Oligomeric tau levels in human brain tissue correlate with Braak stage. Oligomeric tau levels of brain homogenates were probed with the anti-oligomeric tau nanobdy F9T. Light Blue bar: ND Braak stage I-II, no amyloid plaques. Green bar: ND Braak stage I-II slight plaques. Red bar: AD Braak stage III-IV moderate plaques. Dark blue: AD Braak stage V-VI severe plaques. Oligomeric tau levels increase with Braak stage and the presence of amyloid plaques.
**Figure 25****.** Oligomeric a-syn levels in post-mortem human CSF samples readily distinguish PD from AD and ND cases. Oligomeric a-syn levels of CSF samples were determined using the anti-oligomeric a-syn nanobody 10H. Orange bar: PD samples, Maroon: AD samples, Green bar: ND samples High oligomeric a-syn levels were present in PD samples but not AD or ND samples. Samples were diluted 1/50 for analysis.
**Figures 26A-26C****.** Oligomeric Aβ levels in human sera from cognitively normal (ND), AD and PD samples. A) Reactivity with A4 nanobody. B) Reactivity with C6T nanobody. C) Combined oligomeric Aβ levels (A4+C6T). Green bar: ND samples with no plaques, Light pink: ND with moderate plaques. Maroon: AD with moderate plaques. Dark purple: AD with severe plaques. Orange: PD samples. Highest oligomeric Aβ levels are observed in AD samples with moderate plaques, then in AD samples with severe plaques.
**Figure 27****.** Oligomeric a-syn levels in human sera from cognitively normal (ND), AD and PD samples probed with anti-oligomeric a-syn nanobody 10H. Light Orange bar: PD samples with low p129 a-syn reactivity. Medium orange: PD with moderate p129 a-syn reactivity; Dark orange: PD with high p129 a-syn reactivity. Maroon bar: AD samples. Green bar: ND:. Highest oligomeric a-syn levels are observed in early stage PD samples with low levels in late stage PD, AD and ND samples.
**Figure 28****.** A4 and E1 scFv Phage Capture ELISA Using Longitudinal Human Sera Samples.
**Figures 29A-29B****.** 10H scFv readily distinguishes post-mortem PD tissue samples from AD and ND selecting 8 of 9 PD samples and one non-demented cognitively normal (ND) sample; potentially an early stage presymptomatic PD patient.
**Figures 30A-B****.** D5 scFv readily distinguishes post-mortem PD tissue samples from AD and ND selecting 8 of 9 PD samples and one non-demented cognitively normal (ND) sample; potentially an early stage presymptomatic PD patient.
**Figures 31A-B****.** Combined 10H and D5 scFv readily distinguishes post-mortem PD tissue samples from AD and ND selecting all 9 PD samples and one non-demented cognitively normal (ND) sample; potentially an early stage presymptomatic PD patient.
**Figures 32A-B****.** A4 scFv distinguishes post-mortem AD and PD tissue samples from ND selecting 3 of 6 AD and 4 of 9 PD samples.
**Figures 33A-B****.** C6T scFv distinguishes post-mortem AD tissue samples from PD and ND selecting 3 of 6 AD samples.
**Figures 34A-B****.** A4 and C6T scFvs combined distinguishes post-mortem AD and PD tissue samples from ND selecting all 6 AD samples and 4 of 9 PD samples.
**Figures 35A-B****.** 10H scFv readily distinguishes post-mortem PD SERUM samples from AD and ND selecting 5 of 9 PD samples and one AD sample.
**Figures 36A-B****.** D5 scFv readily distinguishes post-mortem PD SERUM samples from AD and ND selecting 6 of 9 PD samples and two AD samples.
**Figures 37A-B****.** 10H and D5 scFv combined readily distinguishes post-mortem PD SERUM samples from AD and ND selecting 6 of 9 PD samples and 2 of 6 AD samples.
**Figures 38A-B****.** A4 scFv readily distinguishes post-mortem AD SERUM samples from PD and ND selecting 5 of 6 AD samples and 2 of 9 PD samples.
**Figures 39A-B****.** C6T scFv readily distinguishes post-mortem AD SERUM samples from PD and ND selecting 3 of 6 AD samples and one non-demented cognitively normal (ND) sample.
**Figs. 40****.** Combined A4 and C6T scFv readily distinguishes post-mortem AD SERUM samples from PD and ND selecting 5 of 6 AD samples and one PD sample.
**Figures 41A-B****.** 10H scFv readily distinguishes post-mortem PD and AD CSF samples from ND selecting 6 of 6 PD samples and 4 of 6 AD samples.
**Figures 42A-B****.** D5 scFv readily distinguishes post-mortem PD CSF samples from AD and ND selecting 5 of 6 PD samples.
**Figures 43A-B****.** Combined 10H and D5 scFv readily distinguishes post-mortem PD CSF samples from AD and ND selecting 6 of 6 PD samples and 2 of 6 AD samples with lower reactivity.
**Figures 44A-B****.** A4 scFv readily distinguishes post-mortem AD CSF samples from ND selecting 6 of 6 AD samples with slight reactivity for 1 ND sample.
**Figures 45A-B****.** C6T scFv distinguishes post-mortem AD CSF samples from ND selecting 3 of 6 AD samples.
**Figures 46A-B****.** Combined A4 and C6T scFvs readily distinguishes post-mortem AD CSF samples from ND selecting 5 of 6 AD samples.

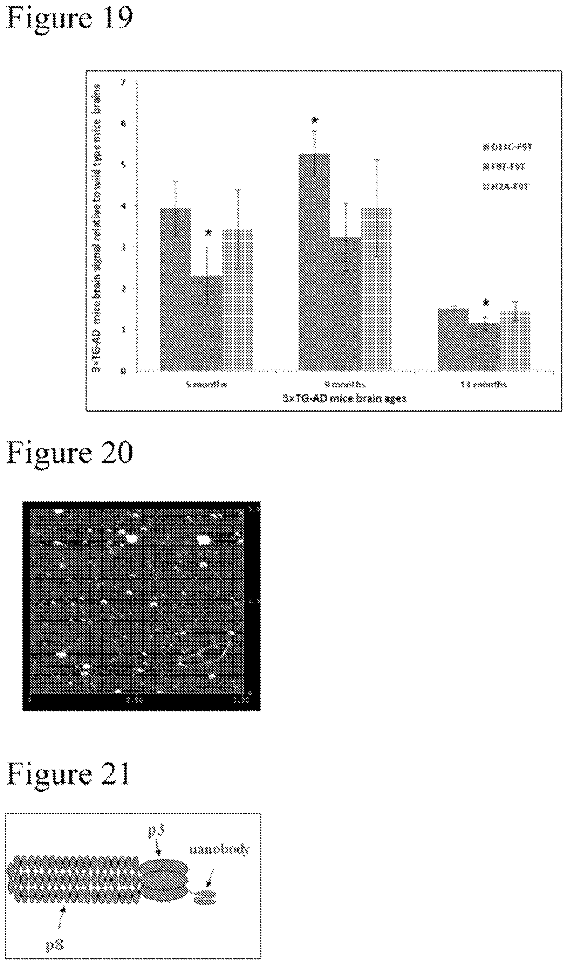

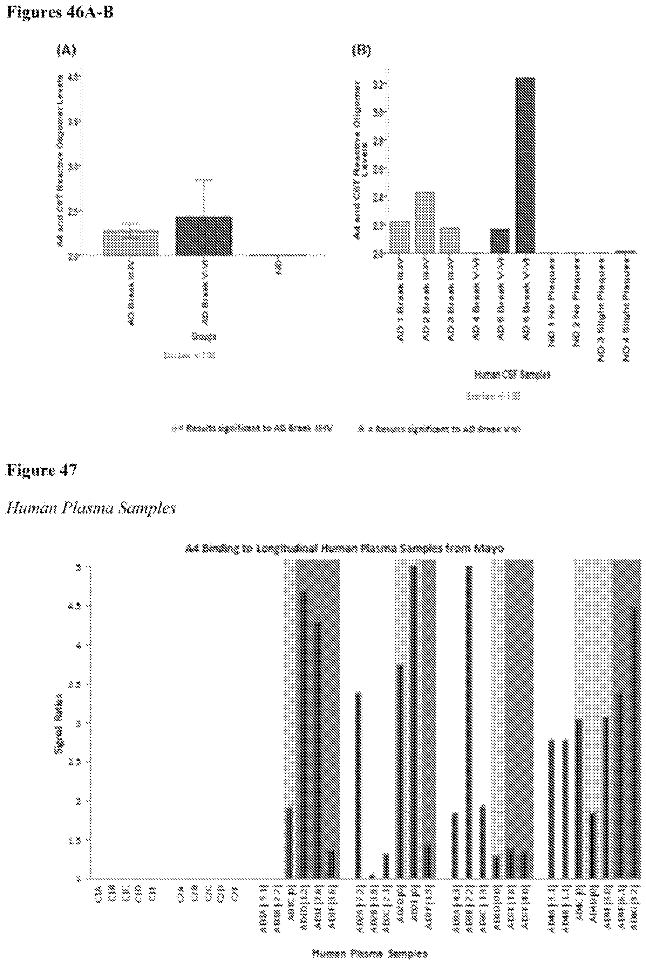

**Figures 47-49****.** Longitudinal analysis of human plasma samples. We analyzed plasma samples taken from 6 different patients over a period of 8-12 years. Initially all the patients were cognitively normal, but 4 of the 6 patients were subsequently diagnosed as having mild-cognitive impairment (MCI) and then all four eventually were diagnosed as having AD. Samples from the six different patients are grouped where samples from the 2 patients that remained cognitively normal are on the left and samples from the 4 patients that converted to AD are on the right. Samples taken after diagnosis of MCI are shaded light purple while samples taken after diagnosis of AD are shaded in dark purple. In Fig 19, A4 scFv clearly distinguishes the 4 patients that converted to AD from the 2 cognitively normal samples. In Fig 20, E1 scFv similarly distinguishes the AD from ND samples, while Fig. 21 shows the combined reactivity of A4 and E1. Impressively, none of the control samples showed any reactivity while almost all of the samples of patients that eventually converted to AD showed significant reactivity not only when diagnosed initially with MCI, but even up to 7 years before diagnosis of MCI when the patients were cognitively normal suggesting the possibility for presymptomatic blood based diagnosis of specific neurodegenerative disease.

### DETAILED DESCRIPTION

The term "biomarker" is generally defined herein as a biological indicator, such as a particular molecular feature, that may affect or be related to diagnosing or predicting an individual's health. A biomarker is a traceable substance that indicates the presence or degree of a biological or pathogenic process (Henley SM, Bates GP, Tabrizi SJ. Biomarkers for neurodegenerative diseases. Curr Opin Neurol. 2005; 18(6): 698-705). Biomarkers are particularly applicable to neurodegenerative diseases as they can provide an early diagnosis at a stage when disease-altering treatments are expected to be most effective. Treating the disease during this initial stage has the potential to circumvent the devastating neurodegeneration characteristic of these diseases. Analogously, biomarkers can monitor the efficiency of a therapy through disease progression. A sensitive method of detection for disease-specific biomarkers that are present during pathogenesis would allow for the study of the toxic mechanisms and metabolic pathways that cause the neurodegenerative diseases and would eventually lead to an early diagnostic method (Sierks et al., 2011).

In certain embodiments, the present invention uses morphology specific nanobody reagents as potential diagnostics for neurodegenerative disease. It has been shown to be possible to distinguish between different neurodegenerative diseases using post-mortem tissue, CSF and serum samples. It has also been shown to be possible to detect Alzheimer's disease in patients using serum samples taken from living patients. Surprisingly, it is shown to be possible to detect a very strong signal in most samples many years before onset of AD and even 2-3 years before diagnosis of mild-cognitive impairment.

The morphology specific nanobodies described here can also be used to monitor progress of different therapeutic interventions. The nanobodies can be used to monitor both CSF and serum samples during a selected treatment, although it is expected that different biomarker sets may distinguish CSF and serum samples in different diseases. Since the nanobodies are antibody fragments, they can be readily incorporated into any existing ELISA platform such as LUMINEX. Nanobodies that recognize toxic protein species present in CSF or blood samples also have potential value as therapeutics that will very selectively target toxic protein aggregates without interfering with monomeric protein function.

For purposes of the diagnostic methods of the invention, an agent may be conjugated to a detecting reagent that facilitates detection of the agent. For example, example, the detecting reagent may be a direct label or an indirect label. The labels can be directly attached to or incorporated into the detection reagent by chemical or recombinant methods.

In one embodiment, a label is coupled to the agent through a chemical linker. Linker domains are typically polypeptide sequences, such as poly gly sequences of between about 5 and 200 amino acids. In some embodiments, proline residues are incorporated into the linker to prevent the formation of significant secondary structural elements by the linker. In certain embodiments, linkers are flexible amino acid subsequences that are synthesized as part of a recombinant fusion protein comprising the RNA recognition domain. In one embodiment, the flexible linker is an amino acid subsequence that includes a proline, such as Gly(x)-Pro-Gly(x) where x is a number between about 3 and about 100. In other embodiments, a chemical linker is used to connect synthetically or recombinantly produced recognition and labeling domain subsequences. Such flexible linkers are known to persons of skill in the art. For example, poly(ethylene glycol) linkers are available from Shearwater Polymers, Inc. Huntsville, Ala. These linkers optionally have amide linkages, sulfhydryl linkages, or heterofunctional linkages.

The detectable labels used in the assays of the present invention to diagnose neurodegenerative disease (e.g., Alzheimer's Disease), these labels are attached to the agent, can be primary labels (where the label comprises an element that is detected directly or that produces a directly detectable element) or secondary labels (where the detected label binds to a primary label, *e.g.,* as is common in immunological labeling). An introduction to labels, labeling procedures and detection of labels is found in Polak and Van Noorden (1997) Introduction to Immunocytochemistry, 2nd ed., Springer Verlag, N.Y. and in Haugland (1996) Handbook of Fluorescent Probes and Research Chemicals, a combined handbook and catalogue Published by Molecular Probes, Inc., Eugene, Oreg. Patents that described the use of such labels include U.S. Pat. Nos. 3,817,837; 3,850,752; 3,939,350; 3,996,345; 4,277,437; 4,275,149; and 4,366,241.

Primary and secondary labels can include undetected elements as well as detected elements. Useful primary and secondary labels in the present invention can include spectral labels such as green fluorescent protein, fluorescent dyes (*e.g*., fluorescein and derivatives such as fluorescein isothiocyanate (FITC) and Oregon Green™, rhodamine and derivatives (*e.g.*, Texas red, tetrarhodimine isothiocynate (TRITC), etc.), digoxigenin, biotin, phycoerythrin, AMCA, CyDyes.TM., and the like), radiolabels (*e.g*., ³H, ¹²⁵I, ³⁵S, ¹⁴C, ³²P, ³³P, etc.), enzymes (*e.g.,* horse radish peroxidase, alkaline phosphatase etc.), spectral calorimetric labels such as colloidal gold or colored glass or plastic (*e.g*. polystyrene, polypropylene, latex, etc.) beads. The label can be coupled directly or indirectly to a component of the detection assay (*e.g.*, the detection reagent) according to methods well known in the art. As indicated above, a wide variety of labels may be used, with the choice of label depending on sensitivity required, ease of conjugation with the compound, stability requirements, available instrumentation, and disposal provisions.

Exemplary labels that can be used include those that use: 1) chemiluminescence (using horseradish peroxidase and/or alkaline phosphatase with substrates that produce photons as breakdown products as described above) with kits being available, *e.g*., from Molecular Probes, Amersham, Boehringer-Mannheim, and Life Technologies/Gibco BRL; 2) color production (using both horseradish peroxidase and/or alkaline phosphatase with substrates that produce a colored precipitate (kits available from Life Technologies/Gibco BRL, and Boehringer-Mannheim)); 3) fluorescence using, *e.g*., an enzyme such as alkaline phosphatase, together with the substrate AttoPhos (Amersham) or other substrates that produce fluorescent products, 4) fluorescence (*e.g*., using Cy-5 (Amersham), fluorescein, and other fluorescent tags); 5) radioactivity. Other methods for labeling and detection will be readily apparent to one skilled in the art.

Where the methods are contemplated to be used in a clinical setting, the labels are preferably non-radioactive and readily detected without the necessity of sophisticated instrumentation. In certain embodiments, detection of the labels will yield a visible signal that is immediately discernable upon visual inspection. One example of detectable secondary labeling strategies uses an antibody that recognizes Aβ amyloid fibrils in which the antibody is linked to an enzyme (typically by recombinant or covalent chemical bonding). The antibody is detected when the enzyme reacts with its substrate, producing a detectable product. In certain embodiments. enzymes that can be conjugated to detection reagents of the invention include, *e.g.*, β-galactosidase, luciferase, horse radish peroxidase, and alkaline phosphatase. The chemiluminescent substrate for luciferase is luciferin. One embodiment of a fluorescent substrate for β-galactosidase is 4-methylumbelliferyl-β-D-galactoside. Embodiments of alkaline phosphatase substrates include p-nitrophenyl phosphate (pNPP), which is detected with a spectrophotometer; 5-bromo-4-chloro-3-indolyl phosphate/nitro blue tetrazolium (BCIP/NBT) and fast red/napthol AS-TR phosphate, which are detected visually; and 4-methoxy-4-(3-phosphonophenyl) spiro[1,2-dioxetane-3,2'-adamantane], which is detected with a luminometer. Embodiments of horse radish peroxidase substrates include 2,2'azino-bis(3-ethylbenzthiazoline-6 sulfonic acid) (ABTS), 5-aminosalicylic acid (5AS), o-dianisidine, and o-phenylenediamine (OPD), which are detected with a spectrophotometer, and 3,3,5,5'-tetramethylbenzidine (TMB), 3,3' diaminobenzidine (DAB), 3-amino-9-ethylcarbazole (AEC), and 4-chloro-1-naphthol (4C1N), which are detected visually. Other suitable substrates are known to those skilled in the art. The enzyme-substrate reaction and product detection are performed according to standard procedures known to those skilled in the art and kits for performing enzyme immunoassays are available as described above.

The presence of a label can be detected by inspection, or a detector which monitors a particular probe or probe combination is used to detect the detection reagent label. Typical detectors include spectrophotometers, phototubes and photodiodes, microscopes, scintillation counters, cameras, film and the like, as well as combinations thereof. Examples of suitable detectors are widely available from a variety of commercial sources known to persons of skill. Commonly, an optical image of a substrate comprising bound labeling moieties is digitized for subsequent computer analysis.

In certain embodiments, the C6 nanobody is targeted specifically to Aβ amyloid fibrils that are characteristic of Alzheimer's Disease. The C6 nanobody composition can be used in a diagnostic assay format to determine the presence of brain-derived SDS-stable Aβ oligomers. A variety of immunodetection methods are contemplated for this embodiment. Such immunodetection methods include enzyme linked immunosorbent assay (ELISA), radioimmunoassay (RIA), immunoradiometric assay, fluoroimmunoassay, chemiluminescent assay, bioluminescent assay, and Western blot, though several others are well known to those of ordinary skill. The steps of various useful immunodetection methods have been described in the scientific literature.

In general, the immunobinding methods include obtaining a sample suspected of containing oligomers, and contacting the sample with a first antibody, monoclonal or polyclonal (in this case C6 nanobody), in accordance with the present invention, as the case may be, under conditions effective to allow the formation of immunocomplexes.

The immunobinding methods include methods for detecting and quantifying the amount of stable Aβ oligomers component in a sample and the detection and quantification of any immune complexes formed during the binding process. Here, one would obtain a sample suspected of containing stable Aβ oligomers, and contact the sample with a capture agent, and then detect and quantify the amount of immune complexes formed under the specific conditions described herein.

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, *i.e.,* to bind to, any antigens present. After this time, the sample-antibody composition, such as a tissue section, ELISA plate, dot blot or western blot, will generally be washed to remove any non-specifically bound antibody species, allowing only those molecules specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. U.S. patents concerning the use of such labels include U.S. Pat. Nos. 3,817,837; 3,850,752; 3,939,350; 3,996,345; 4,277,437; 4,275,149 and 4,366,241. Of course, one may find additional advantages through the use of a secondary binding ligand such as a second antibody and/or a biotin/avidin ligand binding arrangement, as is known in the art.

In certain embodiments, the first antibody (i.e., capture agent) that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two step approach. A second binding ligand, such as an antibody, that has binding affinity for the capture agent is used to form secondary immune complexes, as described above. After washing, the secondary immune complexes are contacted with a third binding ligand or antibody that has binding affinity for the second antibody, again under effective conditions and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

In coating a plate with capture agents, one will generally incubate the wells of the plate with a solution of the capture agent, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein or solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a capture agent to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the patient sample and/or labeled phage specific for the target oligomer with solutions such as BSA, bovine gamma globulin (BGG) or phosphate buffered saline (PBS)/Tween. These added agents also tend to assist in the reduction of nonspecific background.

The "suitable" conditions also mean that the incubation is at a temperature or for a period of time sufficient to allow effective binding. Incubation steps are typically from about 1 to 2 to 4 hours or so, at temperatures preferably on the order of 25° C to 37°C., or may be overnight at about 4°C.

Following all incubation steps in an ELISA, the contacted surface is washed so as to remove non-complexed material. An example of a washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. This may be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (*e.g*., incubation for 2 hours at room temperature in a PBS-containing solution such as PBS-Tween).

After incubation with the labeled antibody, and subsequent to washing to remove unbound material, the amount of label is quantified, *e.g*., by incubation with a chromogenic substrate such as urea, or bromocresol purple, or 2,2'-azino-di-(3-ethyl-benzthiazoline-6-sulfonic acid (ABTS), or H₂O₂, in the case of peroxidase as the enzyme label. Quantification is then achieved by measuring the degree of color generated, *e.g*., using a visible spectra spectrophotometer.

In various aspects of the invention, it will be desirable to further subject patients to more traditional neurodegenerative disease diagnostic approaches. Such general approaches for diagnosis are set out below.

In certain embodiments, the diagnosis of both early (mild) cognitive impairment and AD are based primarily on clinical judgment. However, a variety of neuropsychological tests aid the clinician in reaching a diagnosis. Early detection of only memory deficits may be helpful in suggesting early signs of AD, since other dementias may present with memory deficits and other signs. Cognitive performance tests that assess early global cognitive dysfunction are useful, as well as measures of working memory, episodic memory, semantic memory, perceptual speed and visuospatial ability. These tests can be administered clinically, alone or in combination. Examples of cognitive tests according to cognitive domain are shown as examples, and include "Digits Backward" and "Symbol Digit" (Attention), "Word List Recall" and "Word List Recognition" (Memory), "Boston Naming" and "Category Fluency" (Language), "MMSE 1-10" (Orientation), and "Line Orientation" (Visuospatial). Thus, neuropsychological tests and education-adjusted ratings are assessed in combination with data on effort, education, occupation, and motor and sensory deficits. Since there are no consensus criteria to clinically diagnose mild cognitive impairment, various combinations of the above plus the clinical examination by an experienced neuropsychologist or neurologist are key to proper diagnosis. As the disease becomes more manifest (*i.e.,* becomes a dementia rather than mild cognitive impairment), the clinician may use the criteria for dementia and AD set out by the joint working group of the National Institute of Neurologic and Communicative Disorders and Stroke/AD and Related Disorders Association (NINCDS/ADRDA). On occasion, a clinician may request a head computed tomography (CT) or a head magnetic resonance imaging (MRI) to assess degree of lobar atrophy, although this is not a requirement for the clinical diagnosis.

### Samples

In certain embodiments, the biological fluid is blood, cerebral spinal fluid (CSF), saliva, tears, urine or any bodily fluid. As used herein the term "blood" is defined as whole blood, serum, or plasma. In certain embodiments, the target oligomer to be detected is a toxic oligomeric Aβ, such as trimeric or tetrameric Aβ (early stage AD). In certain embodiments, the target oligomers are a combination of oligomeric Aβ and tau aggregates (later stage AD). In certain embodiments, the target oligomer to be detected is a toxic oligomeric tau, but not Aβ (tauopathies, such as Frontal Temporal Dementia).

In certain embodiments, the target oligomer to be detected is oligomeric a-syn (indicative of Parkinson's Disease). In certain embodiments, the target oligomer to be detected is oligomeric Aβ and a-syn (indicative of Dementia with Lewy Bodies (DLB)).

If levels of toxic Aβ aggregates are much higher than levels of toxic a-syn and tau aggregates, then neurodegeneration is likely precipitated by Aβ and not a-syn or tau, consistent with AD; if levels of toxic tau aggregates are much higher than Aβ or a-syn aggregates, than neurodegeneration is driven by tau aggregation and not Aβ or a-syn, consistent with tauopathies and perhaps later stage AD; and if levels of toxic a-syn aggregates predominate, then neurodegeneration is driven by a-syn and not Aβ or tau consistent with synucleinopathies such as PD.

### Capture Agents

In certain embodiments, the capture agent is an antibody. As used herein, the term "antibody" includes single chain variable fragment (scFv) antibodies (also called a "nanobody"), humanized, fully human or chimeric antibodies, single-chain antibodies, diabodies, and antigen-binding fragments of antibodies (*e.g*., Fab fragments). In exemplary embodiments, the antibody fragment does not contain the constant domain region of an antibody a single chain variable fragment (scFv) antibody. In certain embodiments, the capture agent is scFv-A4 (Zameer, A., et al., Anti-oligomeric Abeta single-chain variable domain antibody blocks Abeta-induced toxicity against human neuroblastoma cells. J Mol Biol, 2008. 384(4): p. 917-28), scFv-C6T (Kasturirangan, S., et al., Isolation and characterization of antibody fragments selective for specific protein morphologies from nanogram antigen samples. Biotechnol Prog, 2013. 29(2): p. 463-71), scFv-10H (Emadi, S., et al., Detecting morphologically distinct oligomeric forms of alpha-synuclein. J Biol Chem, 2009. 284(17): p. 11048-58), scFv-D5 (Emadi, S., et al., Isolation of a human single chain antibody fragment against oligomeric alpha-synuclein that inhibits aggregation and prevents alpha-synuclein-induced toxicity. J Mol Biol, 2007. 368(4): p. 1132-44), or scFv-E1 (Kasturirangan, S., et al., Nanobody specific for oligomeric beta-amyloid stabilizes non-toxic form. Neurobiol Aging, 2012. 33(7): p. 1320-1328).

In certain embodiments, antibody fragments that can be used in the present invention are those listed in Table 1 below:

**Table 1**

| **Antibody Fragment** | **Library source** | **Specificity** | **Assays to validate** | **Applications demonstrated** | **SEQ ID NO** |
|---|---|---|---|---|---|
| A4 | Tomlinson (MRC) | Oligomeric Abeta 3-day aggregates | Dot blot, time course, ELISA, AFM (Note: 3-day oligomeric target is not stable for westerns) | Human AD brain tissue, Human CSF, Mouse AD brain tissue, (Dot blot assays). Immunohistochemistry | amino acid SEQ ID NO:2; nucleic acid SEQ ID NO: 8 |
| E1 | Tomlinson (MRC) | Oligomeric Abeta 1-day aggregates | Dot blot, time course, ELISA, AFM (Note: 1-day oligomeric target is not stable for westerns) | Human AD brain tissue, Human CSF, Mouse AD brain tissue, (Dot blot assays) | amino acid SEQ ID NO:3; nucleic acid SEQ ID NO: 9 |
| C6 | Sheets (UCSF) | Oligomeric Abeta: brain derived | AFM | Human AD brain tissue, Human CSF, Mouse AD brain tissue, (Dot blot assays) | amino acid SEQ ID NO:1; nucleic acid SEQ ID NO: 10 and amino acid SEQ ID NO:15 |
| D5 | Tomlinson (MRC) | Oligomeric a-synuclein 3-day aggregates | Dot blot, time course, ELISA, AFM, western blot analysis | Human PD brain tissue, Human CSF, Mouse PD brain tissue, (Dot blot assays, western blot, Immunohistochemistry with tissue and cells | amino acid SEQ ID NO:4; nucleic acid SEQ ID NO:11 |
| 10H | Tomlinson (MRC) | Oligomeric a-synuclein 7-day aggregates | Dot blot, time course, ELISA, AFM, western blot analysis | Human PD brain tissue, Mouse PD brain tissue, (Dot blot assays, western blot, Immunohistochemistry with tissue and cells | amino acid SEQ ID NO:5; nucleic acid SEQ ID NO: 12 |
| 6E | Tomlinson (MRC) | Fibrillar aggregates (likely not protein specific) | Dot blot, time course, ELISA, AFM | Human PD brain tissue, Human CSF, Mouse PD brain tissue, (Dot blot assays, western blot, Immunohistochemistry with tissue and cells | amino acid SEQ ID NO:6; nucleic acid SEQ ID NO:13 |
| D10 | Tomlinson (MRC) | All forms of a-synuclein | Dot blot, time course, ELISA, AFM, western | Human PD brain tissue, Human CSF, Mouse PD brain tissue, (Dot blot assays, western blot, Immunohistochemistry with tissue and cells | amino acid SEQ ID NO:7; nucleic acid SEQ ID NO:14 |

In certain embodiments, the C6 nanobody has a sequence of SEQ ID NO:1:

EXPIAYGSRWIVITRGPAGHGPGTAAGVGGGLVQPGGSLRLSCAASGFTF
SSYAMSWVRQAPGKGLEWVSAISGSGGSTYYADSVKGRFTISRDNSKNT
LYLQMNSLRAEDTAVYYCAKSYGSVKISCFDYWGQSTLVTVSSGGGGSG
GGGSGGGGSEIVLTQSPDSLAVSLGERATINCKSSQSVLYNSNNKNYLAW
YQQKPGQSPELLIYWASTRESGVPDRFSGSGSGTEFTLTISSLQAEDVAVY
YCQQFYSTPPTFGQGTKLEIKRAAAHHHHHHGAAEQKLISEED

### CDR regions

### AMINO ACID

| | |
|---|---|
| C6T | SYAMS (SEQ ID NO: 16) |
| C6T | AISGSGGSTYYADSVKG (SEQ ID NO: 17) |
| C6T | SYGSVKISCFDY (SEQ ID NO: 18) |
| C6T | KSSQSVLYNSNNKNYLA (SEQ ID NO: 19) |
| C6T | WASTRES (SEQ ID NO: 20) |
| C6T | QQFYSTPPT (SEQ ID NO: 21) |

### DNA

Agctatgccatgagc (SEQ ID NO: 22)
Gctattagtggtagtggtggtagcacatactacgcagactccgtgaagggc (SEQ ID NO: 23)
Agctatggttcagttaaaataagctgctttgactac (SEQ ID NO: 24)
Aagtccagccagagtgttctttacaactccaacaataagaactacttagct (SEQ ID NO: 25)
Tgggcatcaacccgggaatcc (SEQ ID NO: 26)
cagcaattttatagtactcctccgact (SEQ ID NO: 27)

**10H (SEQ ID NO:12)** 
ccatggccgaggtgcagctgttggagtctgggggaggcttggtacagcctggggggtccctgag
actctcctgtgcagcctctggattcacctttagcagctatgccatgagctgggtccgccaggct
ccagggaaggggctggagtgggtctcaaatattagtagtgcagggaaggggctggagtgggtct
caagtattgatgattctggtgcttctacatattacgcagactccgtgaagggccggttcaccat
ctccagagacaattccaagaacacgctgtatctgcaaatgaacagcctgagagccgaggacacg
gccgtatattactgtgcgaaagattctgcttcttttgactactggggccagggaaccctggtca
 
ccgtctcgagcggtggaggcggttcaggcggaggtggcagcggcggtggcgggtcgacggacat
ccagatgacccagtctccatcctccctgtctgcatctgtaggagacagagtcaccatcacttgc
cgggcaagtcagagcattagcagctatttaaattggtatcagcagaaaccagggaaagccccta
agctcctgatctatactgcatccagtttgcaaagtggggtcccatcaaggttcagtggcagtgg
atctgggacagatttcactctcaccatcagcagtctgcaacctgaagattttgcaacttactac
tgtcaacagtctgctgctagtccttctacgttcggccaagggaccaaggtggaaatcaaacggg
cggccgcacatcaccatcaccatcacggggccgcagaacaaaaactcntctcagaagnggatcn
naangggnccg
**D5Q (SEQ ID NO:11)** 
ccatggccgaggtgcagctgttggagtctgggggaggcttggtacagcctggggggtccctgag
actctcctgtgcagcctctggattcacctttagcagctatgccatgagctgggtccgccaggct
ccagggaaggggctggagtgggtctcatcgattggtcagaaggtggtggtacacagtacgcag
actccgtgaagggccggttcaccatctccagagacaattccaagaacacgctgtatctgcaaat
gaacagcctgagagccgaggacacggccgtatattactgtgcgaaacattttgagaattttgac
tactggggccagggaaccctggtcaccgtctcgagcggtggaggcggttcaggcggaggtggca
gcggcggtggcgggtcgacggacatccagatgacccagtctccatcctccctgtctgcatctgt
aggagacagagtcaccatcacttgccgggcaagtcagagcattagcagctatttaaattggtat
cagcagaaaccagggaaagcccctaagctcctgatctatgctgcatccatttgcaaagtgggg
tcccatcaaggttcagtggcagtggatctgggacagatttcactctcaccatcagcagtctgca
acctgaagattttgcaacttactactgtcaacagacgcgtaggccgccttctacgttcggccaa
gggaccaaggtggaaatcaaacgggcggccgcacatcatcatcaccatcacggggccgcagaac
aaaaactcatctcagaagagaatcactagtgcggccgcctgcaggtcgaccata
**6E (SEQ ID NO:13)** 
ttgttattactcgcggcccagccggccatggccgaggtgcagctgttggagtctgggggaggct
tggtacagcctggggggtccctgagactctcctgtgcagcctctggattcacctttagcagcta
tgccatgagctgggtccgccaggctccagggaaggggctggagtgggtctcatatattgctagt
ggtggtgatactacaaattacgcagactccgtgaagggccggttcaccatctccagagacaatt
ccaagaacacgctgtatctgcaaatgaacagcctgagagccgaggacacggccgtatattactg
tgcgaaaggtgcttctgcttttgactactggggccagggaaccctggtcaccgtctcgagcggt
ggaggcggttcaggcggaggtggcagcggcggtggcgggtcgacggacatccagatgacccagt
ctccatcctccctgtctgcatctgtaggagacagagtcaccatcacttgccgggcaagtcagag
cattagcagctatttaaattggtatcagcagaaaccagggaaagcccctaagctcctgatctat
gctgcatcctatttgcaaagtggggtcccatcaaggttcagtggcagtggatctgggacagatt
tcactctcaccatcagcagtctgcaacctgaagattttgcaacttactactgtcaacagagttc
taatgatccttatacgttcggccaagggaccaaggtggaaatcaaacgggcggccgcacatcat
 
catcaccatcacgggngccnnanaacaaaaactcatctcaaannnnntctgaatgggggccnca
tanactgttgaaagttgtttnnaaacctcntacanaaaantcnnttt
**A4 (SEQ ID NO:8)** 
ttgttattactcgcggcccagccggccatggccgaggtgcagctgttggagtctgggggaggct
tggtacagcctggggggtccctgagactctcctgtgcagcctctggattcacctttagcagcta
tcccatgagctgggtccgccaggctccagggaaggggctggagtgggtctcagcgattcagcat
actggtgcgccgacaacttacgcagactccgtgaagggccggttcaccatctccagagacaatt
ccaagaacacgctgtatctgcaaatgaacagcctgagagccgaggacacggccgtatattactg
tgcgaaagcgtttccgccgtttgactactggggccagggaaccctggtcaccgtctcgagcggt
ggaggcggttcaggcggaggtggcagcggcggtggcgggtcgacggacatccagatgacccagt
ctccatcctccctgtctgcatctgtaggagacagagtcaccatcacttgccgggcaagtcagag
cattagcagctatttaaattggtatcagcagaaaccagggaaagcccctaagctcctgatctat
tctgcatcctctttgcaaagtggggtcccatcaaggttcagtggcagtggatctgggacagatt
tcactctcaccatcagcagtctgcaacctgaagattttgcaacttactactgtcaacagcgga
gactgggcctnnnngttcggncaanggancaangtggaaatcaaacgggcggccgcacatcatc
atcaccatcacggggccgcanaacaaaaactcatctcanaanaggatctgaatggggccgcata
nactgttgaanttgtttancaaacnncatacnnnaaattcattt
**E1 (SEQ ID NO:9)** 
ttgttattactcgcggcccagccggcctggccgaggtgcagctgttggagtctgggggaggctt
ggtacagcctggggggtccctgagactctcctgtgcagcctctggattcacctttagcagctat
gccatgagctgggtccgccaggctccagggaaggggctggagtgggtctcatctattcagcctg
agggtaggcggacagcgtacgtagactccgtgaagggccggttcaccatctccagagacaattc
caagaacacgctgtatctacaaatgaacagcctgagagccgaggacacggccgtatattactgt
gcgaaaccgccggagaggtttgactactggggccagggaaccctggtcaccgtctcgagcggtg
gaggcggttcaggcggaggtggcagcggcggtggcgggtcgacggacatccagatgacccagtc
tccatcctccctgtctgcatctgtaggagacagagtcaccatcacttgccgggcaagtcagagc
attagcagctatttaaattggtatcagcagaaaccagggaaagcccctaagctcctgatctatg
ctgcatccagtttgcaaagtggggtcccatcaaggttcagtggcagtggatctgggacagattt
cactctcaccatcagcagtctgcaacctgaagattttgcaacttactactgtcaacagagttac
agtacccctaatacgttcggccaagggaccaaggtggaaatcaaacgggcggccgcacatcatc
atcaccatcacggggccgcagaacaaaaactcatctcanaanaggatctgaatggggccgcata
gactgttgaaagttgtttancaaacctcatacagaaaattcattt
**D10L2 (SEQ ID NO:14)** atggccgaggtgcagctggtggagtctgggggaggcgtggtccagcctgggaggtccctgagac 
tctcctgtgcagcctctggattcaccttcagtagctatggcatgcactgggtccgccaggcccc
aggcaaggggctggagtgggtggcagttatatcatatgatggaagtaataaatactatgcagac
tccgtgaagggccgattcaccatctccagagacaattccaagaacacgctgtatctgcaagtga
acagcctgagagctgaggacacggccgtgtattactgtgcaagaattaatgcgaagtggggcca
aggtaccctggtcaccgtctcgagtggtggaggcggttcaggcggaggtggctctggcggtagt
gcacttgacatccagatgacccagtctccatcctccctgtctgcatctgtaggagacagagtca
ccatcacttgccgggcaagtcagagcattagcagctatttaaattggtatcagcagaaaccagg
gaaagcccctaagctcctgatctatgctgcatccagtttgcaaagtggggtcccatcaaggttc
agtggcagtggatctgggacagatttcactctcaccatcagcagtctgcaacctggagattttg
caacttactactgtcaacagagttacagtaccccgacgttcggcaaggaccaaggtggaaat
caaacgtgcggccgcacatcatcatcaccatcacggggccgcagaacaaaaactcatctcagaa
gaggatct
**C6 (SEQ ID NO:10)**

Ccatggcccaggtacagctgcaggagtcgggggaggcttggtacagcctggggggtccctgaga
ctctcctgtgcagcctctggattcacctttagcagctatgccatgagctgggtccgccaggctc
cagggaaggggctggagtgggtctcagctattagtggtagtggtagcacatactacgcaga
ctccgtgaagggccgattcaccatctccagagacaattccaagaacacgctgtatctgcaaatg
aacagcctgagagctgaggacacggctgtgtattactgtgcgaagagctatggttcagttaaaa
taagctgctttgactactggggccagagcaccctggtcaccgtctcctcaggtggaggcggttc
aggcggaggtggctctggcggtggcggatcggaaattgtgctgacgcagtctccagactccctg
gctgtgtctctgggcgagagggccaccatcaactgcaagtccagccagagtgttctttacaact
ccaacaataagaactacttagcttggtaccagcagaaaccaggacagtctcctgagttgctcat
ttactgggcatcaacccgggaatccggggtccctgaccgattcagtggcagcgggtctgggaca
gaattcactcttaccatcagcagcctgcaggctgaggatgtggcagtttattactgtcagcaat
tttatagtactcctccgactttggccaggggaccaagctggagatcaaacgtgcggccgcaca
tcatcatcaccatcacggggccgcagaacaaaaactcatctcagaagaggatc

### Protein

**10H (SEQ ID NO:5)** 
MAEVQLLESGGGLVQPGGSLRLSCAASGFTFSSYAMSWVRQAPGKGLEWVSNISSAGKGLEWVS
SIDDSGASTYYADSVKGRFTISRDNSKNTLYLQMNSLRAEDTAVYYCAKDSASFDYWGQGTLVT
VSSGGGGSGGGGSGGGGSTDIQMTQSPSSLSASVGDRVTITCRASQSISSYLNWYQQKPGKAPK
LLIYTASSLQSGVPSRFSGSGSGTDFTLTISSLQPEDFATYYCQQSAASPSTFGQGTKVEIKRA
AAHHHHHHGAAEQKLISEEDLNGAA*
**6E (SEQ ID NO:6)** 
MAEVQLLESGGGLVQPGGSLRLSCAASGFTFSSYAMSWVRQAPGKGLEWVSYIASGGDTTNYAD
SVKGRFTISRDNSKNTLYLQMNSLRAEDTAVYYCAKGASAFDYWGQGTLVTVSSGGGGSGGGGS
GGGGSTDIQMTQSPSSLSASVGDRVTITCRASQSISSYLNWYQQKPGKAPKLLIYAASYLQSGV
PSRFSGSGSGTDFTLTISSLQPEDFATYYCQQSSNDPYTFGQGTKVEIKRAAAHHHHHHGAAEQ
KLISEEDLNGAA*
**D5Q (SEQ ID NO:4)** 
MAEVQLLESGGGLVQPGGSLRLSCAASGFTFSSYAMSWVRQAPGKGLEWVSSIGQKGGGTQYAD
SVKGRFTISRDNSKNTLYLQMNSLRAEDTAVYYCAKHFENFDYWGQGTLVTVSSGGGGSGGGGS
GGGGSTDIQMTQSPSSLSASVGDRVTITCRASQSISSYLNWYQQKPGKAPKLLIYAASHLQSGV
PSRFSGSGSGTDFTLTISSLQPEDFATYYCQQTRRPPSTFGQGTKVEIKRAAAHHHHHHGAAEQ
KLISEEDLNGAA*
**A4 (SEQ ID NO:2)** 
MAEVQLLESGGGLVQPGGSLRLSCAASGFTFSSYPMSWVRQAPGKGLEWVSAIQHTGAPTTYAD
SVKGRFTISRDNSKNTLYLQMNSLRAEDTAVYYCAKAFPPFDYWGQGTLVTVSSGGGGSGGGGS
GGGGSTDIQMTQSPSSLSASVGDRVTITCRASQSISSYLNWYQQKPGKAPKLLIYSASSLQSGV
PSRFSGSGSGTDFTLTISSLQPEDFATYYCQQRETGPKAFGQGTKVEIKRAAAHHHHHHGAAEQ
KLISEEDLNGAA*
**E1 (SEQ ID NO:3)** 
MAEVQLLESGGGLVQPGGSLRLSCAASGFTFSSYAMSWVRQAPGKGLEWVSSIQPEGRRTAYVD
SVKGRFTISRDNSKNTLYLQMNSLRAEDTAVYYCAKPPERFDYWGQGTLVTVSSGGGGSGGGGS
GGGGSTDIQMTQSPSSLSASVGDRVTITCRASQSISSYLNWYQQKPGKAPKLLIYAASSLQSGV
PSRFSGSGSGTDFTLTISSLQPEDFATYYCQQSYSTPNTFGQGTKVEIKRAAAHHHHHHGAAEQ
KLISEEDLNGAA*
**D10L2 (SEQ ID NO:7)** 
MAEVQLVESGGGVVQPGRSLRLSCAASGFTFSSYGMHWVRQAPGKGLEWVAVISYDGSNKYYAD
SVKGRFTISRDNSKNTLYLQVNSLRAEDTAVYYCARINAKWGQGTLVTVSSGGGGSGGGGSGGS
ALDIQMTQSPSSLSASVGDRVTITCRASQSISSYLNWYQQKPGKAPKLLIYAASSLQSGVPSRF
SGSGSGTDFTLTISSLQPGDFATYYCQQSYSTPTFGQGTKVEIKRAAAHHHHHHGAAEQKLISE
EDLNGAA*
**C6T (SEQ ID NO:15)**

MAQVQLQESGGGLVQPGGSLRLSCAASGFTFSSYAMSWVRQAPGKGLEWVSAISGSGGSTYYAD
SVKGRFTISRDNSKNTLYLQMNSLRAEDTAVYYCAKSYGSVKISCFDYWGQSTLVTVSSGGGGS

GGGGSGGGGSEIVLTQSPDSLAVSLGERATINCKSSQSVLYNSNNKNYLAWYQQKPGQSPELLI
YWASTRESGVPDRFSGSGSGTEFTLTISSLQAEDVAVYYCQQFYSTPPTFGQGTKLEIKRAAAH
HHHHHGAAEQKLISEEDLNGAA*

In certain embodiments, the antibody fragment comprises amino acid residues 16-292 of SEQ ID NO:1. In certain embodiments, the antibody fragment comprises or consists of amino acid sequence SEQ ID NO:1. In certain embodiments, the antibody fragment is less than 500 amino acids in length, such as between 200-450 amino acids in length, or less than 300 amino acids in length.

### Blocking Agents

In coating a solid substrate (e.g., microwell plate) with a capture agent, one will generally incubate the solid substrate with a solution of the capture agent(s), either overnight or for a specified period of hours. The solid substrate will then be washed to remove incompletely adsorbed material. Any remaining available surfaces of the plate are then "blocked" or "coated" with a nonspecific protein that is antigenically neutral with regard to the test antisera. These include bovine serum albumin (BSA), casein, milk, or solutions of milk powder. The coating allows for blocking of nonspecific adsorption sites on the immobilizing surface and thus reduces the background caused by nonspecific binding of antisera onto the surface. In certain embodiments, the blocking agent is milk. In certain embodiments, the blocking reagent is at a concentration of 0.5% to 10% milk, such as 0.5%, 1%, 2%, 4%, 5% and 10% non-fat dry milk in PBS and 5% and 10% BSA in PBS. In certain embodiments, the blocking reagent is at a concentration of about 2% non-fat dry milk in PBS.

### Incubation Period and Conditions

In certain embodiments, the bound sample is incubated for at least one hour with the capture agent. In certain embodiments, the bound sample is incubated for one to three hours. In certain embodiments, the bound sample is incubated for about two hours.

In certain embodiments, the bound sample is incubated with the capture agent at 30°C to 40°C. In certain embodiments, the bound sample is incubated at about 37°C.

### Labeled Phage Specific for the Target Oligomer

In certain embodiments, the phage specific for the target oligomer is present at a concentration range of 10⁻³ to 10⁻⁵. In certain embodiments, the phage is at a concentration of about 10⁻⁴.

In certain embodiments, the phage is labeled with biotin. For example, the phage is laced by means of Sulfo-NHS biotinylation (amine biotinylation) using the EZ-Link Sulfo-NHS-Biotinylation kit (Thermo Scientific, USA). Briefly, Sulfo-NHS biotin was added in a 20 and 40 fold molar excess to the phage and incubated on ice for 2 hours. The excess biotin was then removed using a zeba spin desalting column (Thermo Scientific, USA). The second type of biotinylation (carboxyl) was performed using the EZ-Link Pentylamine-Biotinylation kit (Thermo Scientific, USA). Pentylamine biotin was added in 20 and 40 fold molar excess to phage along with EDC (1-Ethyl-3-[3-dimethylaminopropyl]carbodiimide hydrochloride). The reactions were incubated at room temperature for 2 hours and the excess biotin removed using the desalting column. The third biotinylation method was performed also using the EZ-Link Pentylamine-Biotinylation kit, except Sulfo-NHS was added to the reaction since this may increase the yield of biotinylated phage. One of our scFvs was also biotinylated using the amine biotinylation kit to compare biotinylated scFv to phage

In certain embodiments, the phage specific for the target oligomer is SRP-phage. As used herein, the term "oligomer" refers to a dimer, trimer, or tetramer.

### Detection Label

In certain embodiments, the biotin is visualized by means of a labeled detection agent. In certain embodiments, the labeled detection agent is avidin operably linked to a label, and in certain embodiments, the avidin is labeled with horseradish peroxidase (HRP).

### Detection Levels

In certain embodiments, the quantity of target oligomer detected is in a fentomolar ratio.

### Detection Kits

Disclosed herein is a kit for detecting one or more target oligomers comprising: (a) a solid support coated with a capture agent specific for the one or more target oligomers; and (b) labelled phage specific for the target oligomer.

In certain embodiments, the kit further comprises a blocking solution. In certain embodiments, the kit further comprises a detection label.

The invention will now be illustrated by the following non-limiting Examples.

### EXAMPLE 1

### DEVELOPMENT OF PHAGE CAPTURE ELISA THAT MEASURES OLIGOMER LEVELS IN ORDER TO DISTINGUISH BETWEEN DIFFERENT NEURODEGENERATIVE DISEASES

Early detection of neurodegenerative diseases would facilitate better treatments and impede disease progression. Oligomeric species of proteins such as alpha-synuclein and abeta are thought to be the toxic species responsible for disease advancement in Parkinson's and Alzheimer's diseases, respectively. Detection of such species is challenging since they are present in low concentrations in the body. We have developed a capture ELISA that is sensitive enough to detect these oligomeric proteins in the brain. Previously isolated scFvs (single-chain variable fragments) that are specific to different neurodegenerative disease were used as the capture antibodies (See, e.g., WO 2012/058308, WO 2012/082237, and US Patent Publication 2012/0230999). Our assay was able to distinguish between healthy and affected individual and between different neurodegenerative diseases. Therefore our new capture ELISA system is a useful tool for the diagnosis of neurodegenerative diseases.

This example details the optimization and development of a novel sandwich ELISA (enzyme-linked immunosorbent assay) that can detect the relatively quantity of a disease-specific antigen.

### Materials and Methods

### Production and Purification of scFvs

The scFvs that binds to different oligomers found in the various neurodegenerative diseases that our lab investigates were produced. Briefly, the scFv was cultured overnight in 2XYT, containing glucose and ampicillin at 37°C. The overnight culture was then transfer to 1L of new expression media and incubated on a shaker until the absorbance O.D. 600 was 0.8. Expression was induced with Isopropyl β-D-1-thiogalactopyranoside (IPTG) and the culture grown overnight. The scFvs were concentrated in a tangential flow filter (Milipore) using a 10 kDa filter membrane (Millipore, Billerica, MA) and the concentrated samples applied to a protein A-Sepharose column (GE healthcare, NJ) for purification using Fast Protein Liquid Chromatography (FPLC). The eluted scFv was dialyzed and stored at -20°C. The purity of the scFvs was estimated using 12% (W/V) SDS-polyacrylamide gels and using Western blotting. Bicinchoninic Acid (BCA) assay (Pierce, USA) was used to determine the concentration of the scFvs.

### Brain Tissue Homogenization

Chopped brain tissue in homogenization buffer (50 mM Tris and 5 mM EDTA at pH 7.5) was sonicated on ice with 10 s bursts followed by 15 second pauses for a total of 5 min using a Fisher sonic dismembrator. All of the samples were centrifuged at 13,000 rpm in a Sorvall SLA-1500 rotor at 4°C for 20 minutes. The supernatants were stored at - 80°C.

### Conditions of Primary Capture scFv

Varying concentrations of the different scFvs were added to a high binding ELISA plate and subjected to different incubation temperatures and lengths of incubation. The plates were washed three times with 0.1% PBS-Tween. The total amounts of protein that remained on the walls of the wells were measured using the BCA kit.

### Phage Production

Phage was produced using the protocol described in the Human Single Fold scFv Libraries I and J manual (MRC Laboratory of Molecular Biology and the MRC Centre for Protein Engineering (Cambridge, UK)). Briefly, our previously isolated clones were cultured overnight in 2XYT containing 100ug/ml ampicillin and 1% glucose. The following day, a 1/100 dilution of the different clones was grown until the OD 600 was between 0.4 and 0.6. 2X10¹¹ of KM13 helper phage was added to the culture and culture incubated at 37°C for 30 minutes without shaking. After spinning at 3000xg for 10 minutes, the pellet was resuspended in 2XYT containing 100ug/ml ampicillin, 50 µg/ml kanamycin and 0.1% glucose and then cultured at 30°C overnight. The cultures were spun for 30 minutes at 3000xg and PEG/NaCl was added to the supernatant. This solution was incubated on ice for 1 hour. The solution was spun, the supernatant discarded and the pellet re-spun briefly. The pellet was dissolved in PBS, spun at 11,600xg and the supernatant stored in glycerol. The newly made phage was titered using TG1 cells to determine its concentration. These phages were used as the detection antibody and should bind all forms of their target protein including monomers and oligomers.

### Biotinylation Protocol

Three different types of biotinylation of phage were performed using different millimoles of biotin. The first was the Sulfo-NHS biotinylation (amine biotinylation) using the EZ-Link Sulfo-NHS-Biotinylation kit (Thermo Scientific, USA). Briefly, Sulfo-NHS biotin was added in a 20 and 40 fold molar excess to the phage and incubated on ice for 2 hours. The excess biotin was then removed using a zeba spin desalting column (Thermo Scientific, USA). The second type of biotinylation (carboxyl) was performed using the EZ-Link Pentylamine-Biotinylation kit (Thermo Scientific, USA). Pentylamine biotin was added in 20 and 40 fold molar excess to phage along with EDC (1-Ethyl-3-[3-dimethylaminopropyl]carbodiimide hydrochloride). The reactions were incubated at room temperature for 2 hours and the excess biotin removed using the desalting column. The third biotinylation method was performed also using the EZ-Link Pentylamine-Biotinylation kit, except Sulfo-NHS was added to the reaction since this may increase the yield of biotinylated phage. One of our scFvs was also biotinylated using the amine biotinylation kit to compare biotinylated scFv to phage.

In order to determine if our phage was indeed biotinylated, the phage was added to an ELISA plate, following by blocking solution (with Bovine Serum Abumin (BSA) or non-fat dry milk) and avidin HRP (Sigma-Aldrich, USA) at 1/10000 dilution. The plates were visualized using 3,3',5,5'-Tetramethylbenzidine (TMB) at 605 nm on a Wallac Victor² microplate reader.

### Determination of the Optimum Biotinylated Phage, Blocking Conditions and Phage Concentration

Purified proteins that may play a role in the different neurodegenerative diseases that we investigate such as alpha-synuclein and Abeta were allowed to aggregate over a certain time period at 37°C. These proteins were used as target antigens in indirect ELISAs to determine which of the biotinylated phages was ideal, and at what dilution as well as the most effective blocking solution. The general indirect ELISA protocol is as follows. The ELISA plates were coated with aggregated proteins for 2 hours at 37°C. The plates were blocked for 1 hour at 37°C followed by 1 hour incubation with the biotinylated phage (bound all forms including monomer and oligomers). Avidin-HRP at 1/5000 dilution was added to the wells for 1 hour at 37°C. The plates were washed three times in between each step and the ELISAs were visualized using TMB.

The best blocking condition was determined using 2 µg/ml of the target antigen followed by varying blocking solutions including 0.5%, 1%, 2%, 4%, 5% and 10% non-fat dry milk in PBS and 5% and 10% BSA in PBS. The best blocking solution was then used along with varying concentrations of the different phages to confirm the best phage dilution. Lastly, we ran an indirect ELISA comparing the phages at 10⁻⁴ dilution with 2 µg/ml of its target antigen and 2% milk as the blocking solution so as to pick the optimum phage.

### Titration of Aggregated Proteins Using Indirect ELISA and TMB

A similar indirect ELISA was carried out as described above. The aggregated target antigen was diluted from nano molar to pico molar range to determine the lowest possible concentration that the 40 mmol carboxyl biotinylated phage can detect. TMB was used as the visualization reagent.

### Phage Capture ELISA using TMB

In the initial phage capture ELISA protocol, 0.3 mg/ml of the capture scFv was added to wells of a high binding ELISA plate for 2 hours at 37°C. The plate was washed three times with 0.1% PBS-Tween. 2% milk was then added to the wells for 1 hour at 37°C and the wash protocol was repeated. The brain samples were added to the wells and incubated overnight at 4°C. The biotinylated phage at 10⁻⁴ dilution was added follow by avidin-HRP (both for 1 hour at 37°C). In the last two steps the washes were increased to four. The plates were visualized using TMB. Results were presented as ratio to no sample control, which is where the absorbance from the wells with samples was divided by the absorbance from the wells with no samples.

### Phage Capture ELISA using Chemiluminescent Kit

0.3 mg/ml of the capture scFv was added to the wells of a high binding ELISA plate for 2 hours at 37°C. The plate was washed three times with 0.1% PBS-Tween. 2% milk was then added to the wells for 1 hour at 37°C and the wash protocol was repeated. The brain samples were added to the wells and incubated for 2 hours at 37°C. The biotinylated phage at 10⁻⁴ dilution was added to wells follow by avidin-HRP (both for 1 hour at 37°C). The plates were visualized using either the SuperSignal ELISA Pico Chemiluminescent Substrate (Thermo Scientific, USA) or the SuperSignal ELISA Femto Maximum Sensitivity Substrate (Thermo Scientific, USA) kit. Results were presented as ratio to the no sample control.

### Synthesis of SRP-phage

The signal recognition pathway (SRP) sequence was generated in a manner similar to what was described by Steiner et al. (Steiner, D., Forrer, P., Stumpp, M. T. & Plückthun, A. (2006). Signal sequences directing cotranslational translocation expand the range of proteins amenable to phage display. Nature Biotechnol. 24, 823-831). To following four oligomers were used to synthesize the SRP for the SRP-phage along with assembly PCR (the lower case letters are where the primers anneal (or attach) to the template DNA, the uppercase letters are sequences that we add on with the PCR reaction):
oDST4N:
   5'- AGCATGAAAAAGATTTggctggcgctggctgg -3' oDST5N: 
5′- TCTTTGTAGTCCGCCGATGCGCTAAACGCTAAAACTAAAccagccagcgccagcc -
3′
oDST6N:
   5'- acaCAGCTagcATGAAAAAGATTT -3'
oDST8N:
   5'- acacaCCATGGCCGGCTGGGCCTCTTTGTAGTCCGCCG -3'

Platinum Pfx DNA polymerase (Invitrogen, USA) with annealing temperatures of 50°C for 1 minute was used in the assembly PCR.

The vector that we cloned the SRP into was generated using the pIT2 vector from the Tomlinson I and J libraries. Briefly, PCR product 1 was generated using primers 1BSAIF 5'-GCCGGTGAGCGTGGTTCTCGCGGTATCATTGC-3' and 2NHEIR 5'-cagcggctagctatgactgtctccttgaaatagaatttgcatgcaagcttg-3' along with the pIT2 vector. PCR product 1 along with primer EWT1N 5' - TTCCGCccatggACCTGGGTAAGAAACTGCTGGAAGCTGCTCGTGCTGGTCAGGACGA CGAAG - 3' and the pIT2 vector was used to generate a large PCR product (PCR product 2). PCR product 2 and the SRP PCR product were digested with NheI and NcoI restriction enzymes (New England Biolabs, USA). After the transformation the new vector obtained was shorter than expected. So this new vector was digested with EcoRI and NcoI along with the original pIT2 vector with one of scFvs of interest. From this new digestion reaction we were able to generate our SMpIT2 vector with one of our scFvs. Next, NcoI and NotI were used to remove the DNA sequence of the current scFv in order to insert different scFvs of interest. The vectors were transformed into TG1 cells and the phage protocol from the Tomlinson I and J libraries was used to produce the SRP-phage.

### Using Normalized Relative Concentrations to Measure Oligomer Levels

In order to create a standard curve to measure the relative concentration of oligomers in the samples, we used one of the samples that had the highest binding to the scFv of interest. This sample was diluted at 1/50, 1/100, 1/200, 1/500, 1/1000 and 1/2000. The signals from the other samples along with the equation of the line from the graph were used to determine the relative dilution of each sample. Then each sample was normalized by dividing each of their relative dilutions by the average relative dilution of the control samples.

### Data Analysis

Statistical analyses for some of the figures were determined using Model I 1-way ANOVA and LSD Post-Hoc analysis (IBM SPSS Statistics 21). Microsoft Excel 2003 and 2010 were used to plot some of the graphs.

### Results

The results for the development of the phage capture ELISA are presented in a general manner since the protocol is applicable to multiple neurodegenerative diseases for which oligomers may play a role in their pathogenesis.

### Production and Purification of scFvs

The scFvs that bind to different oligomers found in the various neurodegenerative diseases that we investigate were produced. Western blot analysis was used to confirm the correct size of the protein produced and SDS-page gel showed the purity of our samples.

### Conditions of Primary Capture scFv

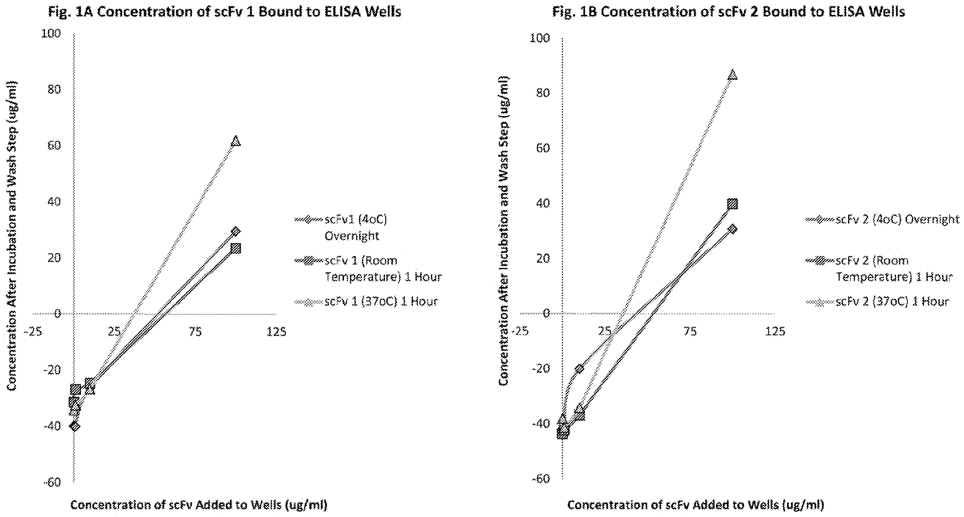

The first step in any capture ELISA is the addition of the capture antibody (in our case scFv) to the wells. Therefore, our first objective was to determine the conditions that would allow maximum saturation of our primary capture scFv to the surface of the wells. Various concentrations of the different scFvs were incubated at 4°C overnight, one hour at room temperature and one hour at 37°C. In this first set of experiments, the amount of scFv added to the wells ranged from 0.1 ug/ml to 100ug/ml. Fig. 1A and Fig. 1B display the results of two of the scFvs that were produced. First, when 100 µg/ml of scFv 1 and 2 were added, the amount of scFv that remained after one hour at 37°C was approximately twice the amount that bound after 4°C overnight or room temperature for one hour (Fig. 1A and 1B). Therefore, 37°C seemed like it may be the best temperature for the antibody incubation.

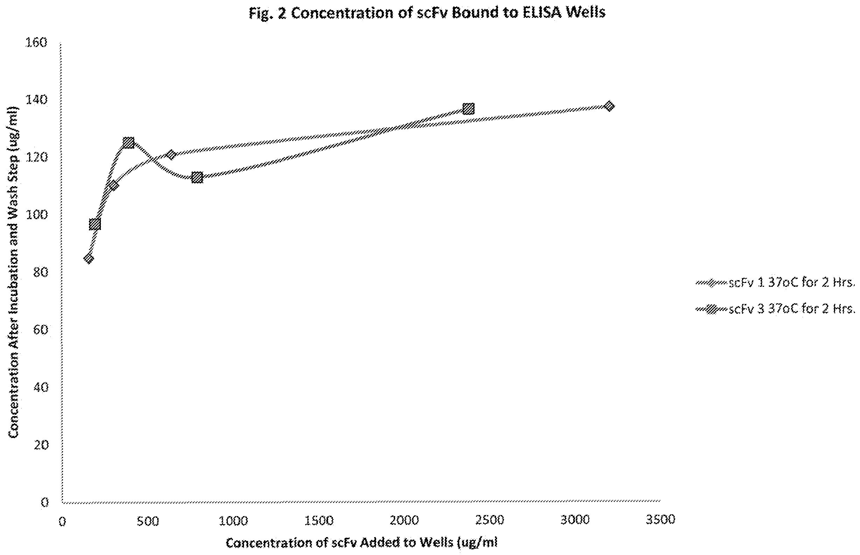

Looking at Fig. 1A and 1B, the lines in the graphs were moving in an upward trend rather than exhibiting saturation even when 100 µg/ml of scFvs were added, indicating that the wells were not saturated. So, in a second experiment, the amount of scFvs added started above 100 µg/ml and increased up to the maximum concentration obtained after scFv production. The scFvs were incubated at 37°C for two hours. The results showed that the wells were saturated somewhere between 100-140ug/ml since as the concentration of the scFvs added increased, the concentration that remain bound to the wells never increased above 140ug/ml (Fig. 2). Since ∼300ug/ml of scFv 1 allowed for over 110 µg/ml of protein to remain in the wells and ∼644 µg/ml of scFv 1 only allowed 121 µg/ml of scFv 1 to remain in the wells, 300 µg/ml of scFv 1 seems like a good concentration to use to saturate the wells (Fig. 2). Similar results were seem for scFv 3 since ∼397 µg/ml allowed for ∼125 µg/ml to remain bound and ∼2385 µg/ml allowed ∼136 µg/ml to bind.

Since, the increase in incubation time from one hour to two hours at 37°C may have also helped with saturating the wells in addition to increasing the scFvs concentration, a third experiment was conducted where the incubation time at 37°C was increased to 3.5 hours (Table 2). Using scFv 1 as an example, after 3.5 hours, 200 µg/ml of scFv 1 resulted in 84 µg/ml of bound scFv. If we look at the two hour incubation at 37°C, ∼160 µg/ml of scFv 1 allowed 85 µg/ml of scFv 1 to bind. There is no difference between 2 hours and 3.5 hours so 2 hours of incubation with the scFvs seems sufficient to allow for saturation of the wells. Comparison of the 3.5 hour room temperature incubation to 3.5 hours at 37°C for 200 µg/ml of scFv 1 demonstrated more binding at 37°C, further supporting 37°C as the optimum incubation temperature. Similar results were seen were seen for scFv 2 and 3 (Table 2). For scFv 2 and 3 the concentration that remained bound after 3.5 hours at 37°C was even a little lower compared to 2 hours at 37°C. Therefore, for the primary capture scFvs the selected conditions for saturation were 300 µg/ml incubated for 2 hours at 37°C.

**Table 2. Different Primary Capture scFvs Binding at Varying Temperatures and Lengths of Incubation**

| **scFvs** | **Incubation Temperatures and Times** | **Original Concentration Added (ug/ml)** | **Concentration Bound (ug/ml)** |
|---|---|---|---|
| | | | |
| **scFv 1** | 37°C for 2 Hrs. | 160 | 85 |
| | 37°C for 3.5 Hrs. | 200 | 84 |
| | Room Temperature for 3.5 Hrs. | 200 | 68 |
| | | | |
| **scFv 2** | 37°C for 2 Hrs. | 227 | 125 |
| | 37°C for 3.5 Hrs. | 200 | 91 |
| | Room Temperature for 3.5 Hrs. | 200 | 55 |
| | | | |
| **scFv 3** | 37°C for 2 Hrs. | 199 | 97 |
| | 37°C for 3.5 Hrs. | 200 | 89 |
| | Room Temperature for 3.5 Hrs. | 200 | 80 |

### Biotinylated Phage vs. scFv as Detection Antibody

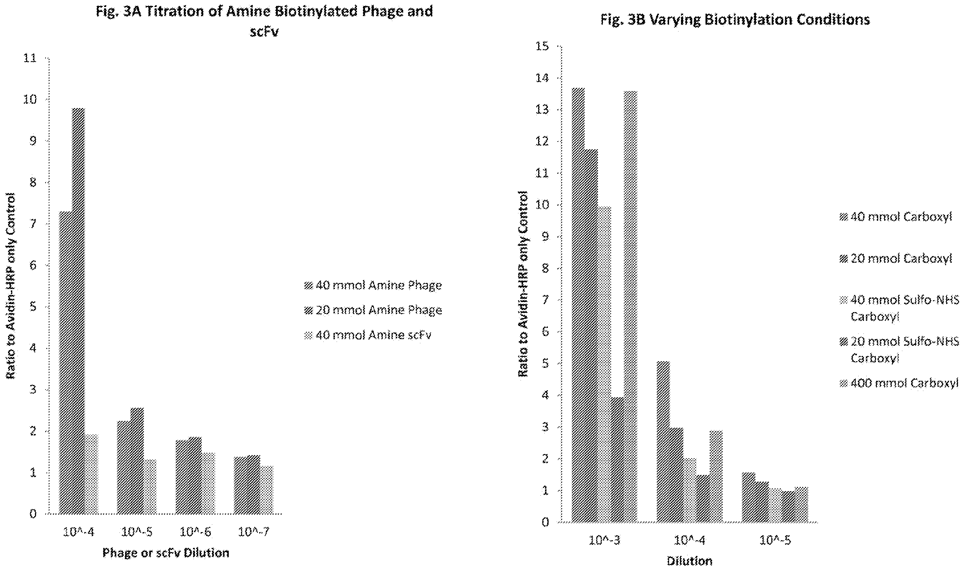

We ran titration analyses on all of the biotinylated phages using avidin-HRP. Our first set of amine biotinylation experiments was used to compare the biotinylated phage to the biotinylated scFv. The phage was biotinylated with 20 and 40 mmol excess of biotin and one of the scFvs was biotinylated with 40 mmol excess. Based on the titration results both the 20 and 40 mmol biotinylated phage gave higher ratios compared to the 40 mmol biotinylated scFv (Fig. 3A) (ratios determined by dividing their absorbance by that of avidin-HRP only control). For the 20 and 40 mmol phage, signals could be detected up to the 10⁻⁶ dilution and for the 40 mmol scFv, signals could be detected up to the 10⁻⁴ dilution (when using 1.5 times the avidin-HRP only control as the cut-off). Using these dilutions, we determine that we could detect up to femto molar concentration of the phage and pico molar concentration of the scFv.

Looking at the other types of biotinylation methods, titration analyses on the 20, 40 and 400 mmol carboxyl biotinylated phages and 20 and 40 mmol Sulfo-NHS carboxyl biotinylated phages also revealed that these biotinylation experiments were successful (Fig. 3B). The 10⁻⁴ dilution seems to give high signals with all the different biotinylation conditions and therefore was a good dilution to use in the next step to determine which of the different biotinylated phages is the best for the ELISA.

### Determination of the Optimum Blocking Conditions, Phage Concentration and Biotinylated Phage

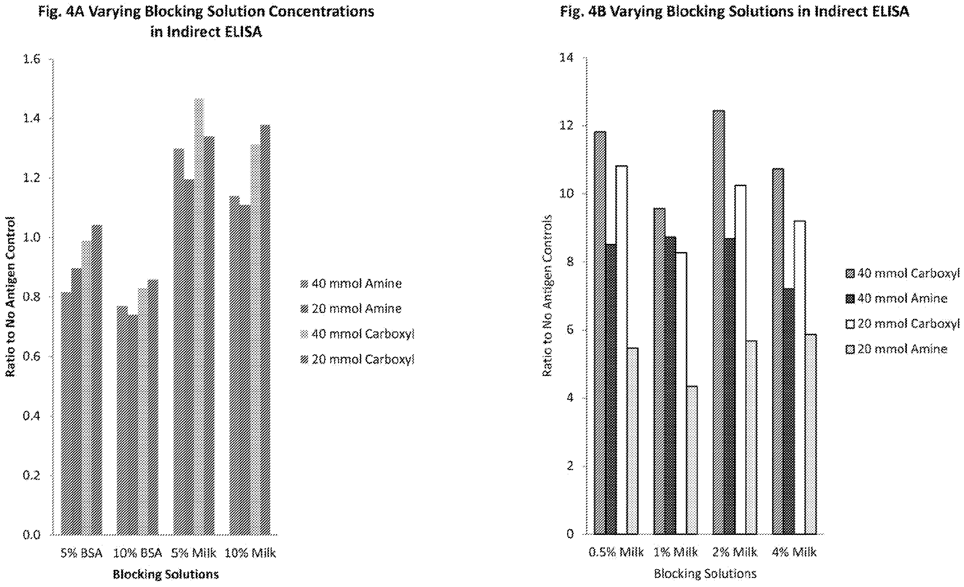

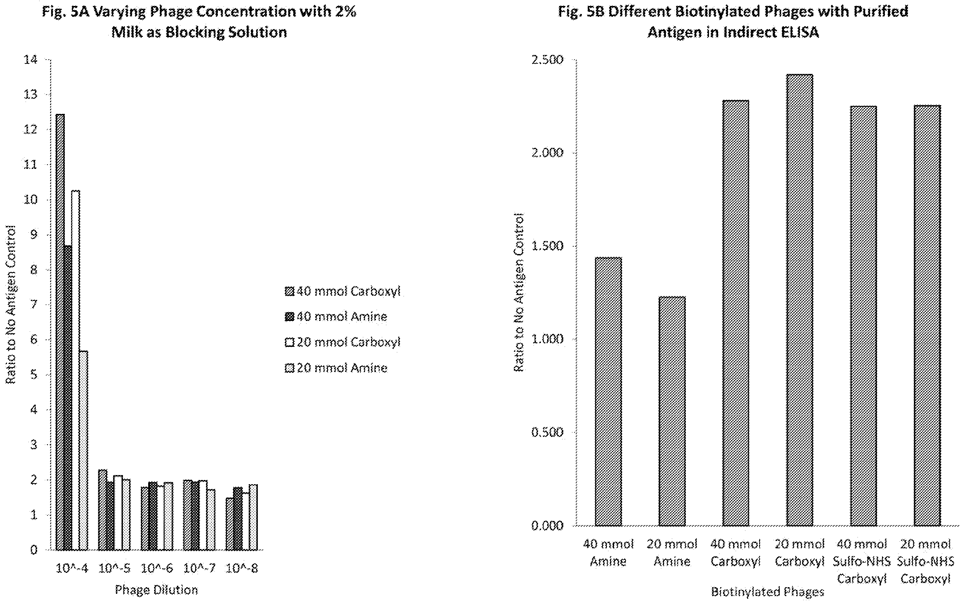

When we initially started to test the phages using their purified target antigen in an indirect ELISA, the background was extremely high that the ratio to no antigen control was lower than 1 (data not shown). To remedy this problem, the target antigen was kept at 2 µg/ml (a concentration that was within the expected detection range of our phage) and the blocking solutions were varied to see if in doing so the wells with no antigens will have much lower signals. We first tried 5% milk, 10% milk, 5% BSA and 10% BSA. As can be seen in Fig. 4A, 5% and 10% milk were the only blocking solutions where our four tested phages gave ratios to no antigen control than were above 1. 5% milk gave slightly better results than 10% milk, however these ratios were still too low and so we tested other blocking solutions. Since milk gave better results than BSA, we decided to only tests additional concentrations of milk and because 5% milk was better than 10% milk we started with concentrations lower than 5%, including 0.5%, 1%, 2% and 4%. Looking at Fig. 4B, the lower milk concentrations seem to do a much better job of blocking the wells since the ratios obtained were considerably higher as compared to those from the blocking solutions used in Fig. 4A. The 2% milk looked the best, especially for the 40 mmol carboxyl biotinylated phage. Therefore, the 2% milk was chosen as the best blocking solution for our ELISA.

Our next objective was to determine the optimum phage concentration. Therefore, using 2% milk as our blocking solution and 2ug/ml of our target antigen, the phages were diluted to include concentrations of 10⁻⁵, 10⁻⁶, 10⁻⁷ and 10⁻⁸. We were able to obtain ratios for all the phages at every dilution that was close or above 1.5 times the no antigen control; many of the ratios approaching 2 times the no antigen control (Fig. 5A). However, because our future intent is to use the phage in a capture ELISA with many different brain, CSF and serum samples (which may have much lower concentrations of our target antigen as compared to our purified samples), we do not want to limit our detection by using too little of the biotinylated phage and therefore 10⁻⁴ dilution seemed like the best choice.

To ascertain which of the six different phages to choose for the capture ELISA, we carried out an indirect ELISA with all of the biotinylated phages at 10⁻⁴ dilution with our target antigen. From this experiment, it can be seen, that the carboxyl biotinylated phages all gave ratios above 2 times the no antigen control when compared to the amine biotinylated phages (Fig. 5B). Something that was noted was that the background was lower when using the carboxyl biotinylated phages, thereby allowing for higher ratios to no sample control. The Sulfo-NHS that should have helped with obtaining high yields of biotinylated phages did not seem to help with obtaining better ratios since the ratios for the 20 and 40 mmol carboxyl only biotinylated phages were slightly higher. When reviewing all the results thus far, the 40 mmol carboxyl biotinylated phage was selected since it gave the best titration results as compared to the other carboxyl biotinylated phages (Fig. 3B) and it gave the highest ratio with the 2% milk at 10⁻⁴ dilution (Fig. 4B).

### Titration of Aggregated Proteins Using Indirect ELISA and TMB

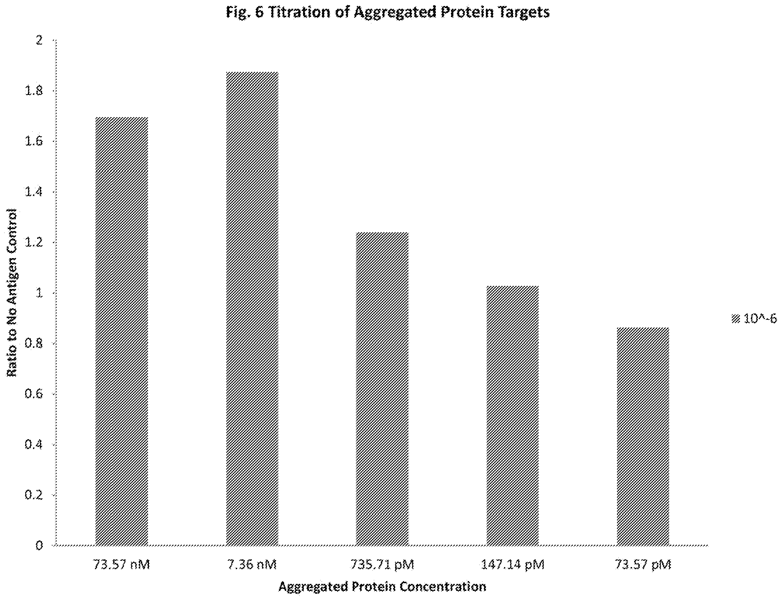

Different concentration of our aggregated target antigens was loaded onto a high binding ELISA plate to determine the lowest concentration detectable by our phage. When looking at all the ratios that were above 1, it appeared that our 40 mmol carboxyl biotinylated phage (which binds to all forms including monomers and oligomers) may be able to detect in the high pico molar range (Fig. 6). Of course, this is based on the assumption that the antigens bound at the concentration at which we loaded them into the wells (which is probably not the case) so in reality our detection system might be better than we assume.

### Phage Capture ELISA using TMB

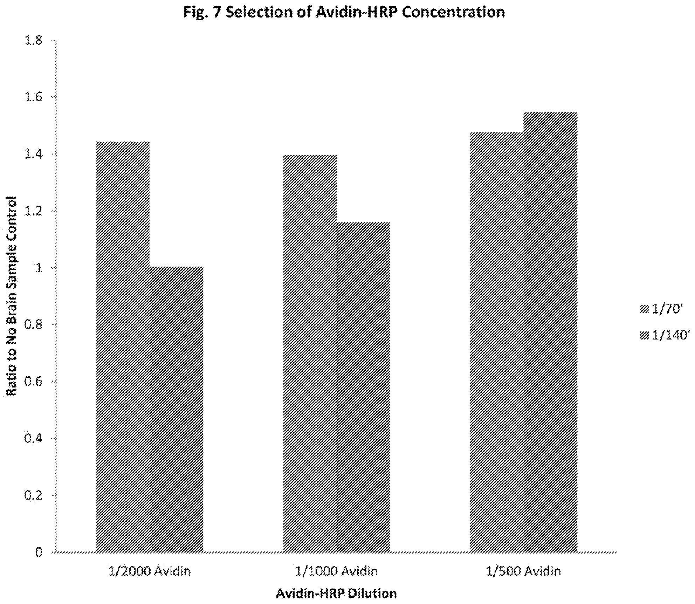

Next, we used the optimized conditions above in a phage capture ELISA. Initially, we tried our new phage capture ELISA protocol on samples from humans with different neurodegenerative diseases and normal individuals. The avidin-HRP concentration that we chose was 1/5000 and a sample concentration of 0.5mg/ml. However, the results were not as expected and the ratios to the no sample control were low (data not shown). Therefore, we decided to vary the avidin-HRP concentration to obtain better results. We tried 1/500, 1/1000 and 1/2000 dilutions of avidin-HRP. Our data showed that increasing the avidin-HRP concentration does help with increasing the ratios to no sample control (Fig. 7). In this ELISA run we had also increased the sample dilutions to 1/70 (0.2 mg/ml) and 1/140 (0.1 mg/ml) since our goal was to develop an ELISA that is very sensitive to low concentrations of oligomers. Looking at the 1/70 dilution, there does not seem to be much of a difference between the 1/2000, 1/1000 and 1/500 dilutions and for the 1/140 sample dilution, the only avidin-HRP dilution that gave a good reading was 1/500. Since the 0.2 mg/ml (1/70) sample dilution gave similar ratios for all the avidin-HRP dilutions, we chose 0.2 mg/ml as the sample concentration and 1/2000 for avidin-HRP.

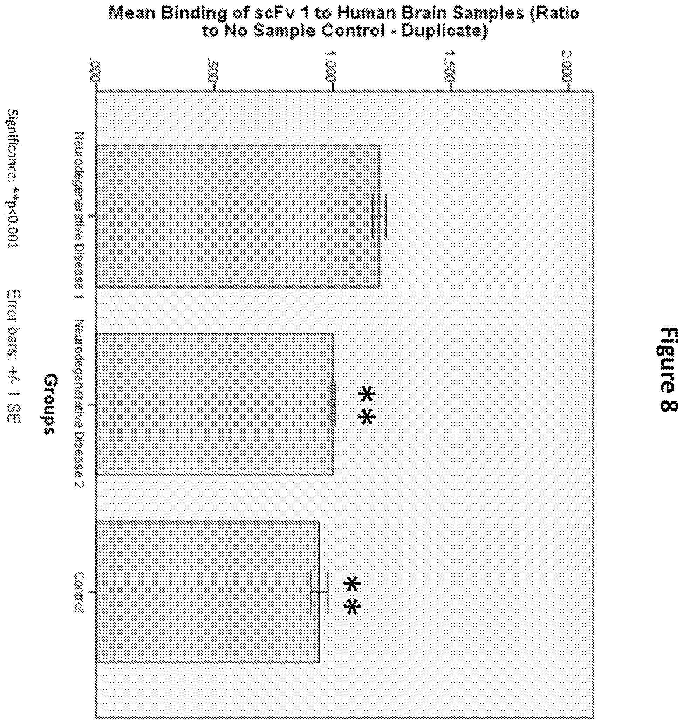

We then retested the human brain samples with our thus far established conditions. There were three different groups that were tested and the group labeling is generic. The first group is labeled as having neurodegenerative disease 1 (9 samples), the second group as having neurodegenerative disease 2 (6 samples) and the third group was the control group (6 samples). Using scFv 1 (which binds oligomers that are specific to neurodegenerative disease 1), ANOVA analysis revealed that there was a significant difference between the groups (Fig. 8). Post-Hoc analysis at p<0.000 showed that there was a significant difference between the group with neurodegenerative disease 1 and the control group and the group with the other neurodegenerative disease 2. This means that our capture ELISA was able to not only distinguish between the control samples and the samples that scFv 1 should bind, but also between different neurodegenerative diseases for which oligomers are part of the pathology.

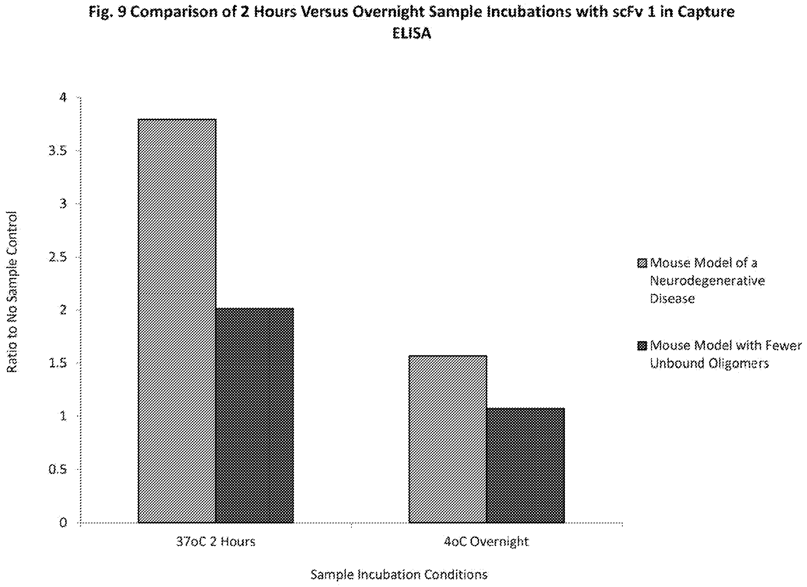

The brain samples above were incubated overnight at 4°C. We then elected to investigate whether or not 4°C overnight was the best choice and so ran another ELISA comparing sample incubations at 4°C overnight and 2 hours at 37°C. Due to limited human samples, we used some brain samples from mice that are a model for the neurodegenerative disease that scFv 1 binds and these same mice treated to have fewer unbound oligomers. We chose one mouse from each group and ran at a concentration of 0.5mg/ml; and even with this small selection, it was clear that 2 hours at 37°C was the better choice (Fig. 9). For the mouse with a neurodegenerative-like disease, its ratio to the no sample control was approximately 3.8 (one of the highest ratios that we had seen so far) and also the difference between the treated and untreated mouse is larger for the 2 hours at 37°C compared to the 4°C overnight. These sample incubation results further demonstrate an improvement with the ELISAs. So in future phage capture ELISAs, we selected 2 hours at 37°C for the sample incubation conditions.

### Indirect and Capture ELISA using Chemiluminescent Kit

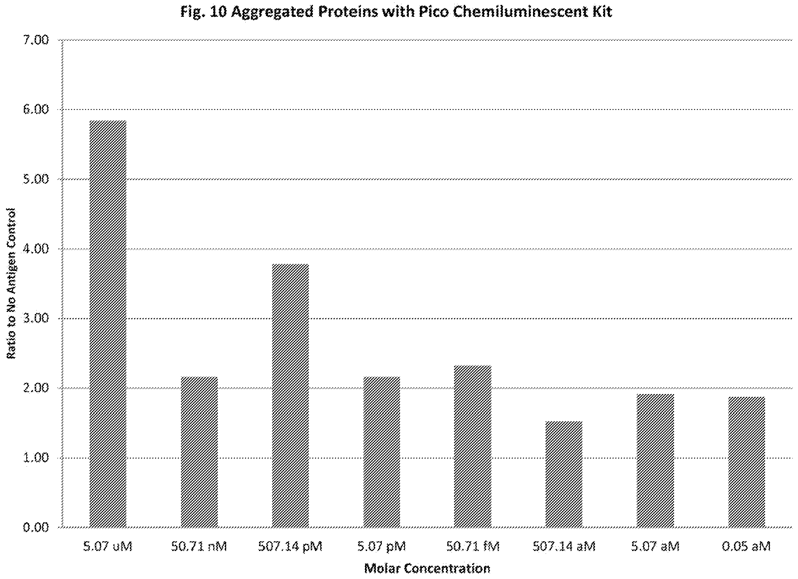

The signals for the mice samples in Fig. 9 looked good, but the average concentration was 0.5 mg/ml (∼1/32 dilution) and our goal concentration was 0.1 mg/ml (∼1/157 dilution). For the human samples that gave significant results in Fig. 8, the ratios to no sample control were still lower than desired and were at a sample concentration of 0.2 mg/ml (not the goal concentration of 0.1 mg/ml). To improve our signals and to be able to lower our sample concentrations, the SuperSignal ELISA Pico Chemiluminescent Substrate (Thermo Scientific, USA) was used instead of TMB. First, an indirect ELISA was used to determine the lowest concentration of purified aggregated proteins (same proteins used in Fig. 6) that could be detected using the Pico kit and the biotinylated phage. If two times the no antigen control is used as a cut-off, then it seems that we were able to detect up to 50.71 femto molar (Fig. 10), possibly even lower. With TMB we were only able to detect up to high pico molar and the ratios to no antigen control were lower compared to the Pico molar kit.

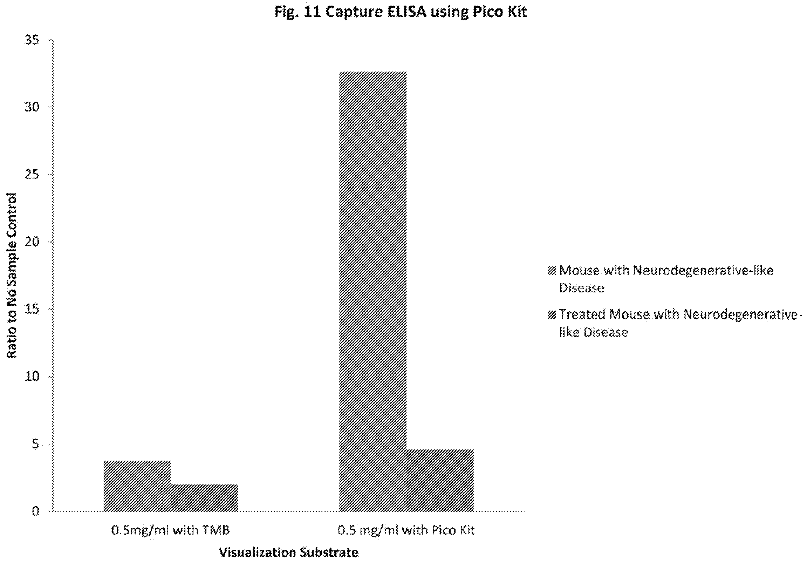

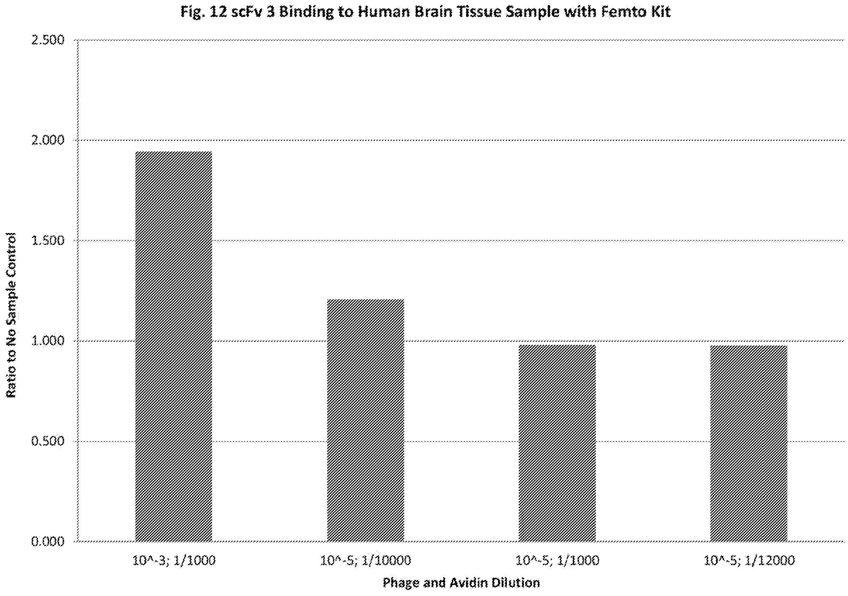

In a phage capture ELISA looking at the two mice brain samples from Fig. 9 at 0.5 mg/ml with the Pico Chemiluminescent kit, it can be seen that not only did the ratios to no sample control increased dramatically, but the difference between the treated and untreated mouse was much larger with the Pico kit (Fig. 11). However, the sample concentration used was still higher than our desired concentration, so we still continued optimizing for detection at a lower concentration. A SuperSignal ELISA Femto Maximum Sensitivity Substrate (Thermo Scientific, USA) kit was also available, so we tried this in a capture ELISA with scFv 3 and a human brain sample at 0.1mg/ml. We also varied the biotinylated phage and avidin-HRP concentrations. Based on the results it seems that the highest ratio was obtained using 10⁻³ phage and 1/1000 avidin-HRP (Fig. 12) and the ratio was still good at 0.1 mg/ml sample concentration.

### Capture ELISA with SRP modified Biotinylated Phage

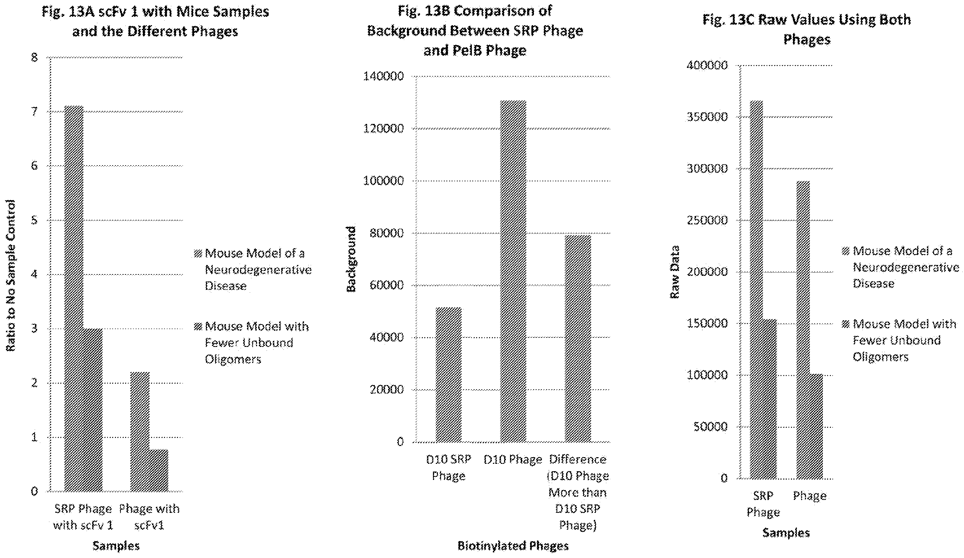

A signal recognition pathway (SRP) that allows for cotranslational translocation of proteins to the periplasm have been shown to help designed ankyrin repeat proteins (DARPins) with the phage display process (Steiner, et al., 2008). This SRP signal may have an effect on the binding of our biotinylated phage to their targets and possibly detect even better at 0.1 mg/ml or lower sample concentration. So in addition to using the 40 mmol carboxyl biotinylated phage, we made phage with the SRP signal and also used 40 mmol carboxyl biotin to biotinylate this SRP-phage. Using the same two mice samples (this time at 0.1mg/ml), we first compared the PelB-phage (original phage) with the SRP-phage. The SRP-phage gave a much higher signal for the mouse with neurodegenerative-like disease when compared to using the PelB-phage and there was also a much larger gap between the treated and untreated mouse (Fig. 13A). Secondly, the SRP-phage had a much lower value for the no sample control as can be seen by the lower background signal and also a huge difference between its background and the background obtained when using the PelB-phage (Fig. 13B). Fig. 13C shows that the raw signal is also higher for the SRP-phage and so the original values being higher along with the lower background is probably what allowed the ratios with the SRP-phage to be higher at the 0.1mg/ml sample concentration.

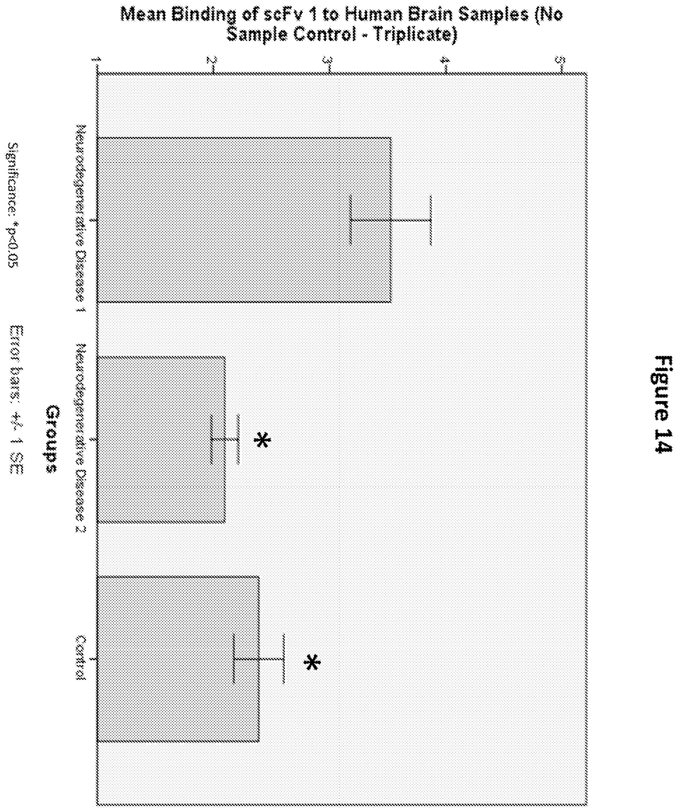

We then ran another capture ELISA using scFv 1, the SRP-phage and the same human brain samples used in Fig. 8. This time we diluted the brain samples to 0.1 mg/ml (∼1/122 dilution). We ran the samples in triplicate to ensure reproducibility of the results and not all of the trials were conducted on the same day. After obtaining the results of each trial, we did ANOVA analysis on each separately and on the average of the three trials to ensure that scFv1 could distinguish between neurodegenerative disease 1 and the other two groups every single time. Our analysis indicated that for each trial scFv 1 gave significantly higher binding to the samples belonging to neurodegenerative disease 1 as compared to the other two groups. The average results of these three trials are shown in Fig. 14. An important detail of Fig. 14 is that at 0.1 mg/ml sample dilution, the average ratio to no sample control for neurodegenerative disease 1 is much higher than the results obtained in Fig. 7, for which we used our initial phage capture ELISA protocol. This means that the sensitivity of our ELISA had increased greatly.

### Using Normalized Relative Concentrations to Measure Oligomer Levels

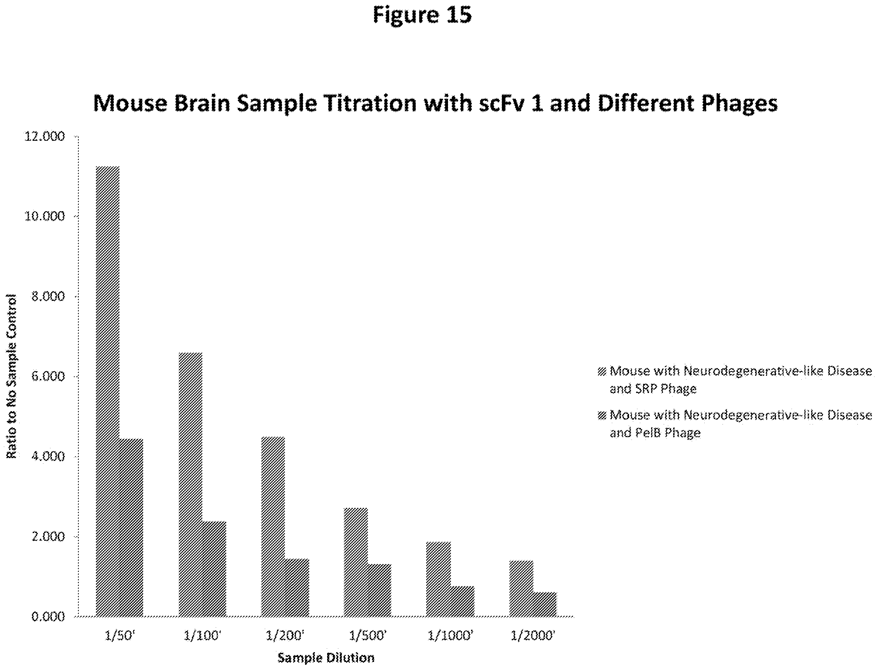

Since it is difficult to accurately measure the level of oligomers in the brain samples, we decided to create a standard to which we can compare all of our samples. We picked the sample that the capture scFv bound the strongest to and made a dilution curve of that sample for every trial that was conducted. Also, the advantage of using the SRP-phage was further demonstrated in Fig. 15, which showed a titration curve of scFv 1 and a mouse sample that binds strongly to this scFv. As can be seen, the SRP-phage gave much higher signal ratios as compare to the PelB-phage for every dilution.

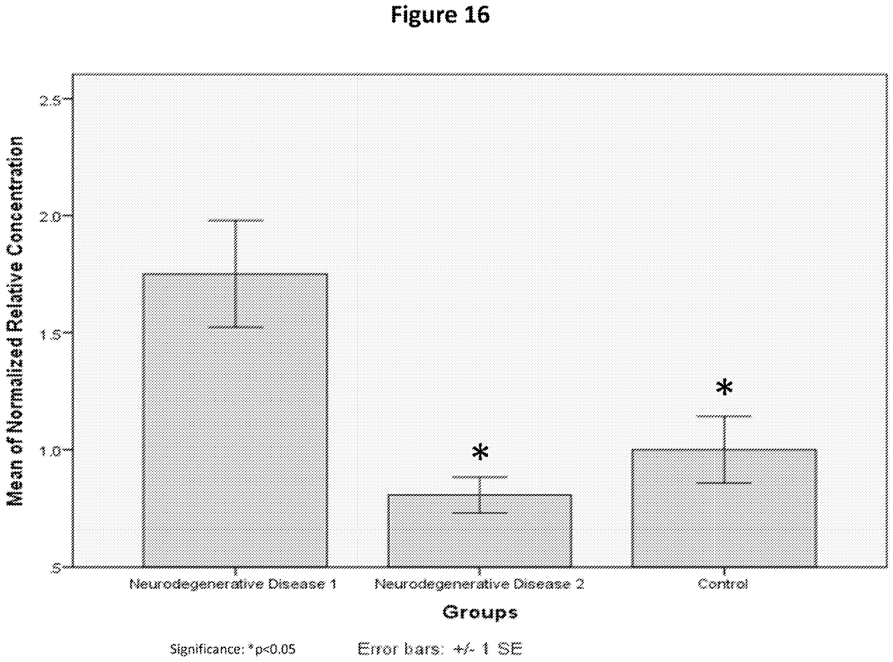

Using the equation of the linear line that was created from our standard curve, we determined the relative dilution of all the other samples. To better illustrate the results, we decided to normalize the relative dilutions to the control samples and to do so, we divided each sample by the average relative dilution of the control group. Fig. 16 shows the average normalized relative dilutions. Even though for some of the optimization procedures small sample sizes (whether mice or humans) were used to establish the most favorable conditions for our phage capture ELISA, these experiments were sufficient since the application of these optimum conditions to larger sample sizes (Fig. 8, Fig. 14 and Fig. 16) gave results that allowed us to clearly distinguish between different neurodegenerative diseases and control samples.

### EXAMPLE 2

### MORPHOLOGY SPECIFIC NANOBODY REAGENTS AS DIAGNOSTICS FOR NEURODEGENERATIVE DISEASE

Biomarkers that can facilitate presymptomatic diagnosis of Alzheimer's disease (AD) and distinguish it from other dementias would be extremely valuable clinical tools. The primary constituents of the two major pathological features of AD, amyloid plaques and neurofibrillary tangles, are respectively, the amyloid beta (Aβ) and tau proteins. Substantial efforts have been expended to identify biomarkers for AD and other neurodegenerative diseases, where the most promising biomarkers for AD to date are variants of Aβ and tau, in particular the 42 amino acid variant of Aβ (Aβ42) and phosphorylated variants of tau. While Aβ and tau biomarkers suffer from a relatively low sensitivity and specificity for diagnosing AD, they still hold great promise for early detection of AD as changes in CSF levels of Aβ42 and tau have been shown to occur well before symptoms develop, up to 25 years earlier for Aβ42.

While CSF Aβ and tau levels correlate with AD, the vast majority of studies have focused on detection of non-toxic monomeric forms of Aβ42 and phosphorylated tau rather than on detection of the actual toxic protein species responsible for neurodegeneration. Both Aβ and tau can exist in a variety of different forms and aggregate morphologies and numerous studies indicate that specific oligomeric forms of both Aβ and tau are involved in neuron degeneration and spread of toxicity, and can interfere with important functions such as long term potentiation. Therefore a more powerful and sensitive diagnosis for AD and other dementias would be to specifically detect the individual protein species that are involved in disease onset and progression. Because misfolded and aggregated variants of Aβ and tau are intimately involved in the progression of AD, detection of specific variants of these proteins in CSF and/or serum has great promise for an early definitive diagnosis of AD and for following progression of the disease or effectiveness of different therapeutic regimens.

In a parallel manner, misfolded toxic oligomeric variants of the protein alpha-synuclein (a-syn) have been correlated with the onset and progression of Parkinson's disease (PD) and related synucleinopathies so detection of the relevant toxic oligomeric a-syn species should facilitate early diagnosis of synucleinopathies such as PD and Dementia with Lewy Bodies (DLB) and help distinguish these diseases from AD. Recent evidence has suggested that cytoplasmic misfolding and aggregation of TAR DNA binding protein 43 (TDP-43) associates with the pathology observed in a high percentage of FTD and ALS cases and more recently in other neurodegenerative diseases including AD and traumatic brain injury. TDP-43 is also prone to misfold and form aggregate species, where disease associated TDP-43 mutations similar to Aβ and a-syn aggregate more readily. Therefore selected toxic aggregated variants of Aβ, tau, a-syn and TDP-43 all have value as early and sensitive diagnostic biomarkers to distinguish different neurodegenerative diseases.

Quantification of serum and CSF levels of the actual toxic protein species involved in disease onset and progression should provide a much more sensitive and powerful set of biomarkers for early detection and staging of neurodegenerative diseases such as AD. Detection of different toxic variants of Aβ, tau, a-syn and TDP-43 in serum and CSF provide a powerful tool to facilitate early diagnosis of different neurodegenerative diseases, for example presence of toxic oligomeric Aβ aggregates are indicative of early AD, oligomeric Aβ and tau aggregates indicate a later stage AD, only oligomeric tau indicate a tauopathy such as Frontal Temporal Dementia, oligomeric a-syn indicate PD, and oligomeric Aβ and a-syn indicate DLB.

While detection of these specific protein aggregate species has great promise, such studies have not been feasible due to the low concentrations of the target aggregate species in CSF and particularly serum samples and the poor specificity of reagents for the different aggregated species. To overcome this problem, we have developed novel protocols that enable us to generate reagents that very selectively recognize individual protein morphologies and we also developed a simple novel sandwich ELISA that enables femtomolar or better detection of target antigens in biological samples. Here we will utilize a panel of such antibody based (nanobody) reagents that selectively recognize different toxic aggregated species of Aβ, tau, a-syn and TDP-43 to demonstrate that the presence of specific toxic aggregate proteins species in serum and CSF are very selective biomarkers for distinguishing AD from other dementias.

There are several unique aspects that distinguish our approach for identifying serum and CSF biomarkers for AD from many previous attempts and strongly increase our chances of succeeding. A fundamental difference in the current approach compared to previous studies is the detection of specific toxic forms of individual proteins that are correlated with disease progression. The presence of oligomeric Aβ has been correlated with AD and memory dysfunction, the presence of tau aggregates has been correlated with AD and other tauopathies, the presence of oligomeric a-syn with PD, and the presence of oligomeric TDP-43 with FTD, ALS and AD. Aggregation of one protein such as Aβ may promote aggregation of other proteins including tau or a-syn (Clinton, L.K., et al., Synergistic Interactions between Abeta, tau, and alpha-synuclein: acceleration of neuropathology and cognitive decline. J Neurosci. 30(21): p. 7281-9; Masliah, E., et al., beta -Amyloid peptides enhance alpha -synuclein accumulation and neuronal deficits in a transgenic mouse model linking Alzheimer's disease and Parkinson's disease. Proc Natl Acad Sci U S A, 2001. 25: p. 25), so quantifying the presence of specific aggregate species of Aβ, tau, a-syn and TDP-43 allows identification of which protein aggregate species are primarily responsible for toxicity and neurodegeneration. Therefore the ability to identify the source of toxic protein aggregation provides a powerful tool to facilitate an accurate and early diagnosis of a particular neurodegenerative disease such as AD. For example, if levels of toxic Aβ aggregates are much higher than levels of toxic a-syn and tau aggregates, then neurodegeneration is likely precipitated by Aβ and not a-syn or tau, consistent with AD; if levels of toxic tau aggregates are much higher than Aβ or a-syn aggregates, than neurodegeneration is driven by tau aggregation and not Aβ or a-syn, consistent with tauopathies and perhaps later stage AD; and if levels of toxic a-syn aggregates predominate, then neurodegeneration is driven by a-syn and not Aβ or tau consistent with synucleinopathies such as PD. Significant presence of more than one aggregate species, such as presence of Aβ and tau may be indicative of a later stage of AD and presence of Aβ and a-syn may be indicative of a neurodegenerative disease such as DLB. Presence of different toxic forms of these proteins may also facilitate staging different diseases. The key to enable such selective detection of toxic protein aggregates is the availability of reagents that specifically recognize individual protein aggregate species. We have developed innovative technologies in our lab to do exactly that, to both generate and characterize recombinant antibody fragments (nanobodies) that selectively bind specific protein morphologies. We developed a novel Atomic Force Microscopy (AFM) based biopanning technology that enables us to isolate nanobodies against specific protein forms (Barkhordarian, H., et al., Isolating recombinant antibodies against specific protein morphologies using atomic force microscopy and phage display technologies. Protein Eng Des Sel, 2006. 19(11): p. 497-502). Utilizing this technology we isolated nanobodies that recognize different areas of monomeric Aβ and a-syn (Emadi, S., et al., Inhibiting Aggregation of alpha-Synuclein with Human Single Chain Antibody Fragments. Biochemistry, 2004. 43(10): p. 2871-2878; Zhou, C., et al., A human single-chain Fv intrabody blocks aberrant cellular effects of overexpressed alpha-synuclein. Mol Ther, 2004. 10(6): p. 1023-31; Liu, R., et al., Single chain variable fragments against beta-amyloid (Abeta) can inhibit Abeta aggregation and prevent abeta-induced neurotoxicity. Biochemistry, 2004. 43(22): p. 6959-67; Zameer, A., et al., Single Chain Fv Antibodies against the 25-35 Abeta Fragment Inhibit Aggregation and Toxicity of Abeta42. Biochemistry, 2006. 45(38): p. 11532-9), fibrillar Aβ and a-syn (Barkhordarian, H., et al., Isolating recombinant antibodies against specific protein morphologies using atomic force microscopy and phage display technologies. Protein Eng Des Sel, 2006. 19(11): p. 497-502; Marcus, W.D., et al., Characterization of an antibody scFv that recognizes fibrillar insulin and beta-amyloid using atomic force microscopy. Nanomedicine, 2008. 4(1): p. 1-7), two different toxic oligomeric a-syn species (Emadi, S., et al., Isolation of a human single chain antibody fragment against oligomeric alpha-synuclein that inhibits aggregation and prevents alpha-synuclein-induced toxicity. J Mol Biol, 2007. 368(4): p. 1132-44; Emadi, S., et al., Detecting morphologically distinct oligomeric forms of alpha-synuclein. J Biol Chem, 2009. 284(17): p. 11048-58), three different toxic oligomeric Aβ species (Zameer, A., et al., Anti-oligomeric Abeta single-chain variable domain antibody blocks Abeta-induced toxicity against human neuroblastoma cells. J Mol Biol, 2008. 384(4): p. 917-28; Kasturirangan, S., et al., Nanobody specific for oligomeric beta-amyloid stabilizes non-toxic form. Neurobiol Aging, 2010. **In press;** Kasturirangan, S., et al., Isolation and Characterization of a Nanobody that Selectively Binds Brain Derived Oligomeric Beta-Amyloid Biotechnol Prog, 2013. **(In press))** and a toxic oligomeric tau species.

The different oligomer specific nanobodies do not show cross-reactivity, so the nanobodies binding oligomeric Aβ do not bind oligomeric a-syn and vice versa, nor do they cross react with monomeric or fibrillar aggregates species. We have shown that each of the different aggregate species recognized by the different nanobodies naturally occur in human AD or PD tissue, and that the nanobodies can be used to distinguish between AD, PD and healthy brain tissue, and block toxicity of different aggregate species. Therefore we have a unique panel of well characterized and highly selective reagents that can be used to detect the presence of key aggregated protein forms in CSF and serum samples that are associated with different neurodegenerative diseases including AD. To detect the low concentrations of the toxic oligomeric protein species, we have also devised a simple novel sandwich ELISA.

We incorporated a phage display version of the nanobodies as the detection agent in the ELISA which utilizes the several thousands of copies of the phage coat protein as a simple means to amplify the detection signal by several orders of magnitude. The phage can be readily generated by *E. coli* at minimal cost and represents a promising method to significantly enhance sensitivity of the ELISA. In addition to the unique reagents and ELISA protocol, other advantages of this proposal over previous studies include the use of postmortem CSF and serum from cases with *neuropathologically confirmed* AD, neuropathologically confirmed non-AD dementia and control subjects, all of whom have had standardized serial cognitive and neuromotor assessment during life as well as postmortem neuropathologic examination. A large subject set is available, allowing for an accurate determination of sensitivity and specificity.

While many neurodegenerative diseases have overlapping clinical symptoms and cellular and biochemical mechanisms such as an increase in inflammatory markers, and aggregation of similar proteins, the reagents we propose to study here have well defined specificity and selectivity for individual toxic Aβ, tau and a-syn forms and should facilitate specific diagnoses of AD and other neurodegenerative diseases. We are also developing additional reagents against other AD associated oligomeric forms of tau and against oligomeric TDP-43. Once the specificity of each of these reagents is validated, the new reagents will also be incorporated into this study expanding the number of disease specific biomarkers we can detect. Because this set of reagents selectively recognizes toxic protein variants associated with different neurodegenerative diseases, this study should help identify specific toxic species involved in different stages of AD and help to provide a pre-symptomatic diagnosis to distinguish AD from other neurodegenerative diseases including frontotemporal dementia and other tauopathies, and Parkinson's disease, Dementia with Lewy bodies and other synucleinopathies.

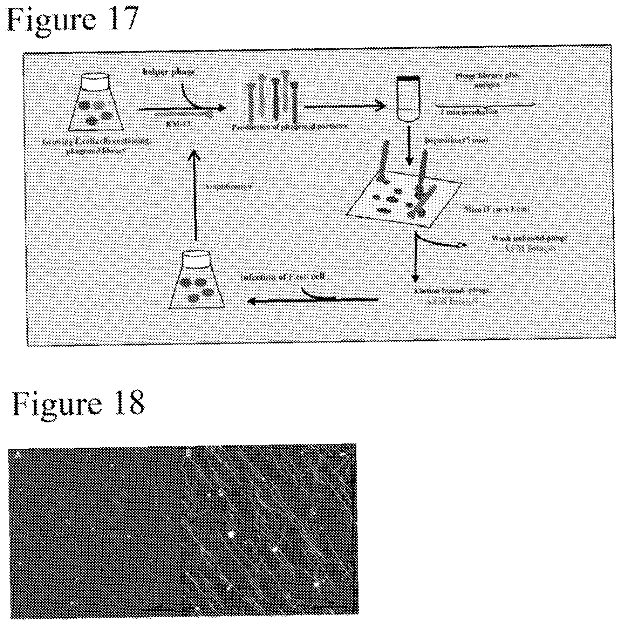

***A) Isolation of nanobodies to specific protein morphologies.*** We developed a novel biopanning technology that combines the imaging capability of Atomic Force Microscopy (AFM) with the diversity of antibody libraries (Fig. 17) (Barkhordarian, H., et al., Isolating recombinant antibodies against specific protein morphologies using atomic force microscopy and phage display technologies. Protein Eng Des Sel, 2006. 19(11): p. 497-502). This capability has enabled us to isolate nanobody reagents to a variety of different morphologies of key proteins involved in neurodegenerative diseases including Aβ, a-syn and tau. We have continued to refine our panning protocols to facilitate isolation of reagents against targets that are available in limited amounts, that cannot be purified or that are unstable. We have published results reporting isolation of nanobodies against monomeric, fibrillar and two different oligomeric forms of a-syn, and monomeric, fibrillar and three distinctly different oligomeric Aβ species including an AD brain derived variant (Barkhordarian, H., et al., Isolating recombinant antibodies against specific protein morphologies using atomic force microscopy and phage display technologies. Protein Eng Des Sel, 2006. 19(11): p. 497-502; Emadi, S., et al., Inhibiting Aggregation of alpha-Synuclein with Human Single Chain Antibody Fragments. Biochemistry, 2004. 43(10): p. 2871-2878; Zhou, C., et al., A human single-chain Fv intrabody blocks aberrant cellular effects of overexpressed alpha-synuclein. Mol Ther, 2004. 10(6): p. 1023-31; Liu, R., et al., Single chain variable fragments against beta-amyloid (Abeta) can inhibit Abeta aggregation and prevent abeta-induced neurotoxicity. Biochemistry, 2004. 43(22): p. 6959-67; Zameer, A., et al., Single Chain Fv Antibodies against the 25-35 Abeta Fragment Inhibit Aggregation and Toxicity of Abeta42. Biochemistry, 2006. 45(38): p. 11532-9; Emadi, S., et al., Isolation of a human single chain antibody fragment against oligomeric alpha-synuclein that inhibits aggregation and prevents alpha-synuclein-induced toxicity. J Mol Biol, 2007. 368(4): p. 1132-44; Emadi, S., et al., Detecting morphologically distinct oligomeric forms of alpha-synuclein. J Biol Chem, 2009. 284(17): p. 11048-58; Zameer, A., et al., Anti-oligomeric Abeta single-chain variable domain antibody blocks Abeta-induced toxicity against human neuroblastoma cells. J Mol Biol, 2008. 384(4): p. 917-28; Kasturirangan, S., et al., Nanobody specific for oligomeric beta-amyloid stabilizes non-toxic form. Neurobiol Aging, 2010. **In press;** Kasturirangan, S., et al., Isolation and Characterization of a Nanobody that Selectively Binds Brain Derived Oligomeric Beta-Amyloid Biotechnol Prog, 2013. **(In press))**.

The different specificities of each nanobody can be readily observed when the nanobody is expressed on the surface of a filamentous bacteriophage and antibody/antigen complexes are imaged by AFM. Using this technique, we showed that the C6T nanobody selectively binds a brain derived oligomeric Aβ variant, but not in vitro generated species, E1 binds 1 day preaggregated in vitro Aβ, but not brain derived Aβ species or 3 day preaggregated in vitro Aβ, and A4 binds 3 day preaggregated in vitro Aβ, but not 1 day or brain derived samples. We include additional negative panning steps during the panning process to remove nonspecific and undesired binding activities, so virtually all clones isolated after only a single round of panning specifically recognize the target antigen, so we can readily isolate various morphology specific ligands using only picograms of target. We have also developed AFM based protocols to characterize ligand binding, so we can not only isolate morphology specific ligands with only minimal material, but we can characterize binding specificity with limited material as well.

In addition to the nanobodies against oligomeric forms of Aβ and a-syn, we also have used this panning protocol to isolate nanobodies against an oligomeric morphology of tau generated by synthetically cross-linking tau monomers in collaboration with Oligomerix (New York, NY). We isolated nanobodies that selectively recognize a toxic trimeric tau form but not monomeric tau (Fig. 18). We used three different nanobodies specific for the trimeric tau species to determine if the oligomeric tau was present in a 3xTg mouse model of AD (Oddo, S., et al., Triple-transgenic model of Alzheimer's disease with plaques and tangles: intracellular Abeta and synaptic dysfunction. Neuron, 2003. 39(3): p. 409-21). All three nanobodies reacted with the brain tissue showing that high concentrations of oligomeric tau are already present in 5 month old brain tissue (Fig. 19), well before the neurofibrillary tangles are detected providing further evidence that detection of oligomeric protein species is a promising tool for early detection of neurodegenerative disorders.

We are currently performing similar panning studies to isolate nanobodies that selectively bind aggregated forms of TDP-43 that are present in ALS and FTD brain tissue but not healthy brain tissue. We have a number of potential clones that show reactivity to TDP-43 aggregates derived from diseased tissue but not healthy TDP-43 forms (Fig. 20). Once the binding specificity of these nanobodies has been verified, these nanobodies will be included in the assays as well.

Therefore we have nanobody reagents to monomeric, fibrillar and three different oligomeric Aβ species, to monomeric, fibrillar and two different oligomeric a-syn species, and to oligomeric tau. These nanobodies are a powerful resource to probe human CSF and serum samples for the presence of aggregated protein variants that are indicative of different stages of AD. We have also developed a simple yet very sensitive sandwich ELISA assay using these nanobodies that can be used to detect and quantify the levels of oligomeric protein variants in human brain tissue, CSF and serum samples as described below.

***B) Sandwich ELISA with sub-Femtomolar sensitivity.*** While Aβ, tau and a-syn are present in CSF and serum at low nanomolar concentrations (Lesne, S., et al., A specific amyloid-beta protein assembly in the brain impairs memory. Nature, 2006. 440(7082): p. 352-7; Walsh, D.M., et al., The role of cell-derived oligomers of Abeta in Alzheimer's disease and avenues for therapeutic intervention. Biochem Soc Trans, 2005. 33(Pt 5): p. 1087-90), we expect that the concentrations of different oligomeric Aβ, tau and a-syn species in CSF will be significantly lower, therefore a sensitive assay is needed to quantitatively determine the concentrations of each different target. We previously showed that we could detect oligomeric protein variants in post-mortem human CSF samples using our nanobodies in conjunction with an electronic biosensor and that we could distinguish between cognitively normal (ND), PD and AD samples (Sierks, M.R., et al., CSF Levels of oligomeric alpha-synuclein and beta-amyloid as biomarkers for neurodegenerative disease. Integrative Biology, 2011. 3(12): p. 1188-96).

To facilitate quantitative analysis of multiple samples in a format that can be replicated in multiple labs, we alternatively developed a simple yet sensitive sandwich ELISA to detect low concentrations of target antigens in human samples. In a typical sandwich ELISA, one antibody is used to capture the target antigen and a second detection antibody, often conjugated with a fluorescent marker or enzyme such as horseradish peroxidase (HRP) is used to quantify the amount of bound target. For oligomeric protein variants we can either use the same nanobody to both capture and detect the target species since the oligomeric aggregates have multiple binding sites on each target molecule, or use an oligomeric specific nanobody to capture the target and a non-morphology selective nanobody to detect the bound target. To amplify the detection signal for increased sensitivity we use a phage displayed version of the detection antibody similar to what we use in the biopanning studies. A single detection antibody is thus connected to a phage particle which contains over 2000 copies of the gp8 coat protein (Fig. 21). Biotinylation of the coat proteins enables several thousand-fold amplification of the detection signal compared to using the nanobody alone. A streptavidin/HRP complex is used to bind the biotinylated phage and a colorimetric or chemilluminescence substrate to quantify the bound target. We can detect antigen at well below femtomolar concentrations using a chemilluminescence substrate. Using this sandwich ELISA, we could readily detect the presence of oligomeric protein aggregates not only in human brain tissue homogenates, but also in human CSF and serum samples as well, as will be described in the following section.

***C) Morphology Specific Nanobodies to Distinguish and Stage AD using Human Tissue. CSF and Serum samples.** Human Tissue.* We have shown that our morphology specific nanobodies distinguish human post-mortem ND, AD and PD brain tissue samples, where the oligomeric a-syn specific nanobodies selectively label PD tissue and the oligomeric Aβ specific nanobodies selectively label AD tissue. To demonstrate the potential value of morphology specific nanobodies in diagnosing neurodegenerative diseases we analyzed well characterized brain samples from the middle temporal gyrus of six non-demented (ND), six AD and nine Parkinson's (PD) patients using the sandwich ELISA protocol described above. We analyzed post-mortem human brain tissue using two anti-oligomeric Aβ nanobodies, A4 and C6T, an anti-oligomeric a-syn nanobody 10H, and an anti-oligomeric tau nanobody F9T. Samples were prepared as described (Emadi, S., et al., Isolation of a human single chain antibody fragment against oligomeric alpha-synuclein that inhibits aggregation and prevents alpha-synuclein-induced toxicity. J Mol Biol, 2007. 368(4): p. 1132-44; Emadi, S., et al., Detecting morphologically distinct oligomeric forms of alpha-synuclein. J Biol Chem, 2009. 284(17): p. 11048-58).

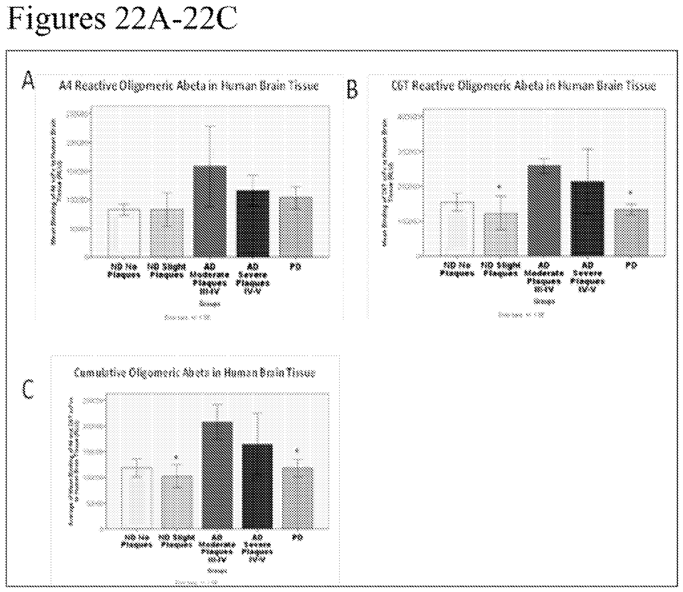

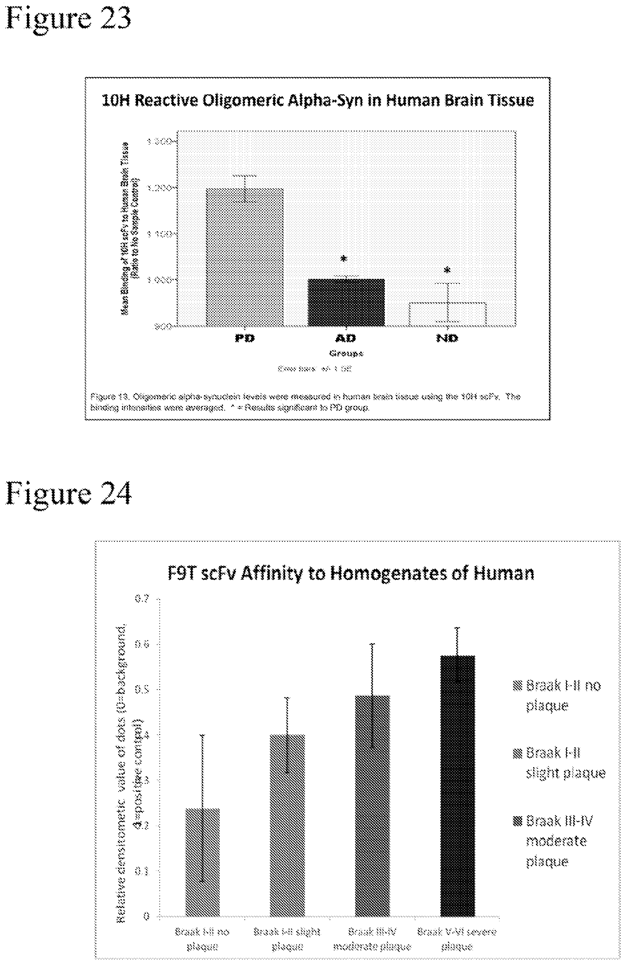

Protein concentrations of each sample were normalized and equal volumes used where the samples were diluted on average around 1/70 for analysis. When assaying oligomeric Aβ levels, both the A4 and C6T nanobodies could readily distinguish between AD and PD and ND samples and could stage AD as higher levels of oligomeric Aβ were observed in AD samples with moderate plaques compared to AD samples with severe plaques (Fig. 22). What is particularly exciting about the data is that the anti-oligomeric Aβ nanobodies readily distinguish between ND and AD cases even in samples that have similar amyloid plaque loads. Therefore while amyloid plaque deposition does not correlate well with AD disease progression, presence of specific oligomeric Aβ species may correlate very well. The oligomeric Aβ levels decrease as the disease progresses from moderate to severe plaque loads. When the same samples were analyzed for presence of oligomeric a-syn using the 10H anti-oligomeric a-syn nanobody, all the PD samples reacted strongly, whereas the AD and ND samples showed only background levels (Fig. 23) indicating that oligomeric a-syn levels can be used to distinguish between PD, AD and ND samples.

Finally, when the same samples were analyzed for the presence of oligomer tau using the F9T nanobody again we could readily distinguish between AD and ND samples with oligomeric tau levels increasing with Braak stage (Fig. 24). Of particular interest, the anti-oligomeric tau antibody can distinguish between Braak stage I and II samples with and without the presence of plaques, showing higher oligomeric tau levels in brain samples that also contain the presence of slight amount of amyloid plaques. These results suggest that the presence of oligomeric tau may be a valuable early diagnostic for AD and further validate that morphology specific reagents can be a powerful tool to detect dementia associated with AD. When the results of the post-mortem human brain tissue sample analysis with all the nanobodies are combined, we have a very powerful set of reagents that can not only readily distinguish between different neurodegenerative diseases, but potentially can stage these diseases as well. While the analysis of post-mortem brain tissue with our nanobodies is a very powerful tool, for diagnostic applications we need to be able to make similar distinctions in CSF and ideally serum samples as well. The next section presents the results obtained using post-mortem CSF and serum samples.

*Human Post-mortem CSF and serum.* We have also shown that our morphology specific nanobodies can recognize oligomeric aggregates in postmortem human CSF and serum samples and can distinguish between diseased and cognitively normal samples. CSF and serum samples were also obtained from the world's only brain bank with a consistently-run rapid autopsy protocol, with a median postmortem interval of 3.2 hours for all 1450-plus brains in the collection. It is recognized that postmortem CSF changes must be distinguished from changes due to disease and therefore the postmortem interval must be considered as a possible source of variability, however, in practice we have found that over our very short post-mortem interval (PMI) range (all samples are under 6 hours PMI) more than 90% of measured proteins and peptides show no correlation with PMI. Living donors receive standardized yearly medical, neurological and neuropsychological assessments. The neurologic examination includes a psychiatric inventory (BEHAVE-AD), an ADL instrument, the Mini-Mental Status Examination (MMSE) and a functional assessment (GDS, FAST and BCRS). In addition to cognitive evaluation, all donors receive a standardized neurological evaluation tailored to detect and quantify movement disability. All subjects are also scored using the Unified Parkinson's Disease Rating Scale (UPDRS). Standardized neuropathologic examinations are performed on every brain postmortem. All cases are also classified according to CERAD criteria (Mirra, S.S., et al., The Consortium to Establish a Registry for Alzheimer's Disease (CERAD). Part II. Standardization of the neuropathologic assessment of Alzheimer's disease. Neurology, 1991. 41(4): p. 479-86) for neuritic plaque and tangle abundance, and all cases are assigned a Braak tangle stage (Braak, H. and E. Braak, Neuropathological stageing of Alzheimer-related changes. Acta Neuropathol (Berl), 1991. 82(4): p. 239-59).

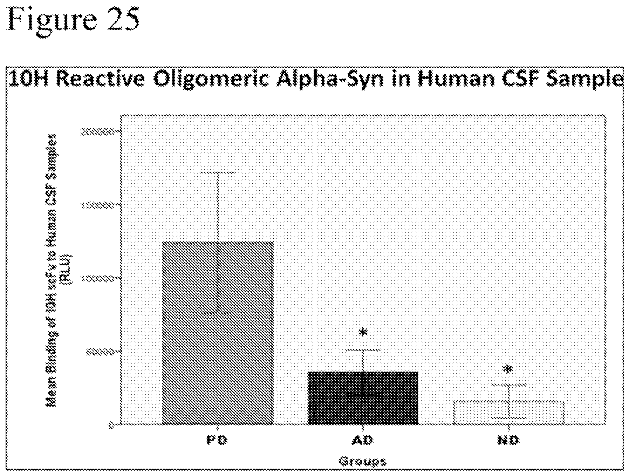

We analyzed a small set of post-mortem CSF samples from the BSHRI tissue bank with our morphology specific nanobodies. We previously showed that our nanobodies in conjunction with an electronic biosensor could detect the presence of oligomeric Aβ and a-syn in these CSF samples and could readily distinguish between CSF samples from age matched cognitively normal (ND), AD and PD patients (Sierks, M.R., et al., CSF Levels of oligomeric alpha-synuclein and beta-amyloid as biomarkers for neurodegenerative disease. Integrative Biology, 2011. 3(12): p. 1188-96). We used the sandwich ELISA described above to analyze additional post-mortem CSF samples for presence of different oligomeric protein variants using the anti-oligomeric a-syn nanobody 10H. CSF samples were diluted 1/50 for analysis. We could again readily distinguish PD from AD and ND samples using the 10H nanobody (Fig. 25).

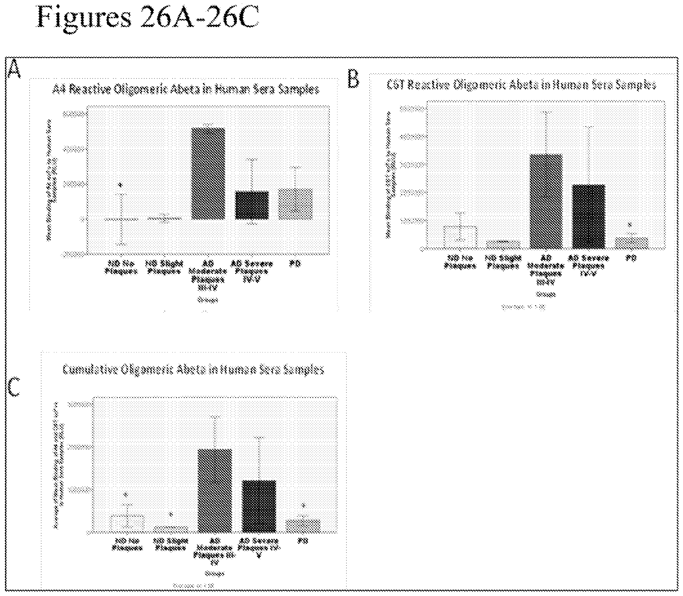

We also analyzed a set of post-mortem serum samples from the BSHRI tissue bank with our morphology specific nanobodies. The serum samples were from the same patients used for the brain tissue studies described above in Figs 22-24. We used the sandwich ELISA described above to determine the concentrations of oligomeric variants of Aβ and a-syn in the serum samples. Serum samples were diluted 1/50 for analysis. When testing for oligomeric Aβ species using the C6T and A4 nanobodies, we essentially mirrored the results obtained with the brain tissue homogenates as we could readily distinguish between AD and PD and ND samples (Fig. 26). Again very impressively, we could very readily distinguish between the AD and ND serum samples even when similar amyloid plaque loads were present and again higher levels of oligomeric Aβ were observed in samples with moderate plaques and lower levels in samples with severe plaques.

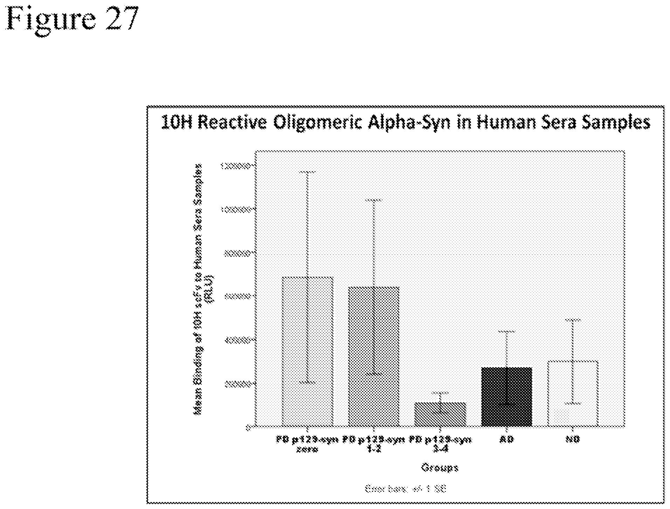

Similarly, when testing serum samples for oligomeric a-syn species using the 10H nanobody, we could readily distinguish between PD and AD and ND samples (Fig. 27) where once again higher signals were obtained with samples from early disease stages and lower signals with later stages. These very exciting preliminary results indicate that we can detect the presence of specific toxic oligomeric variants of Aβ and a-syn in both CSF and serum samples and that the levels of the different oligomeric proteins correlate very well with specific neurodegenerative diseases and with different stages of disease. Here we propose to expand the preliminary data presented here to look at a larger set of data and to include samples that have mixed pathology including Aβ, tau and a-syn deposits.

### Experimental Design and Methods

***A. Characterization of samples.** CSF and serum samples.* CSF and serum samples from the BSHRI Brain Bank are derived from subjects with neuropathologically confirmed diagnoses including at least 40 with AD and 40 non-demented age-similar controls. Samples are distributed by Braak stage: 0-II, III-IV and V-VI and contain varying amounts of amyloid plaques (zero plaques, sparse plaques, moderate plaques and frequent plaques) and a-syn pathology (sparse, moderate and frequent). All subjects have a PMI less than 5 hours. Detailed clinical and neuropathological data are available on these subjects, including MMSE, CERAD neuritic plaque density scores, Braak tangle stage and regional Lewy-type alpha-syncleinopathy density scores.

*Analysis of samples by ELISA.* Samples are analyzed using a sandwich ELISA as described above in the *supporting data section.* For analysis of oligomeric Aβ, we use three different anti-oligomeric nanobodies, A4, E1 and C6T as capture antibodies and a phage displayed version of H1v2 (Yuan, B., P. Schulz, and M.R. Sierks, Improved Affinity Selection of an scFv Antibody Fragment Against -amyloid Using Phage Display Technology and Off-rate Based Selection. Electronic Journal of Biotechnology, 2006. 9(2): p. 171-175), a non-morphology specific Aβ nanobody as a detection antibody. For analysis of oligomeric a-syn, we use two different anti-oligomeric a-syn nanobodies, 10H and D5 as capture antibodies and a phage displayed version of D10 (Emadi, S., et al., Inhibiting Aggregation of alpha-Synuclein with Human Single Chain Antibody Fragments. Biochemistry, 2004. 43(10): p. 2871-2878) a non-morphology specific a-syn nanobody as a detection antibody. For analysis of oligomeric tau, we use the anti-oligomeric tau nanobodies, F9T, H2A and D11C as capture antibodies and a phage displayed version of F9T as a detection antibody. The same reagents and assays were used to generate the preliminary data presented above. CSF and serum samples are diluted 1/50 for analysis.

*Statistical Analyses.* All assays are performed at least in triplicate to ensure reproducibility. Statistical analysis is performed in one-way ANOVA and LSD post hoc to compare means of ND versus AD groups.

### EXAMPLE 3

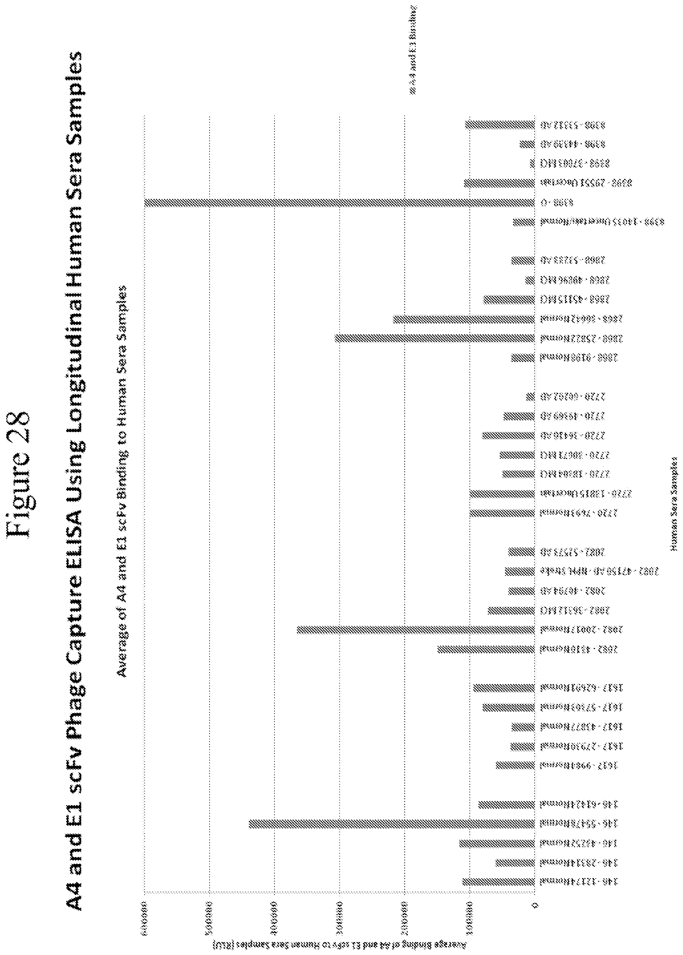

We analyzed serum samples taken over a period of 5-8 years from patients that started out cognitively normal. Four of the patients after the initial samples were subsequently diagnosed with mild cognitive impairment (MCI) and then AD several years after that. The control patients were cognitively normal at the time, although they may still convert to MCI or AD in the future. We suspect one of the controls will convert to MCI soon. We analyzed six samples, two controls and 4 that converted to mci and AD. We analyzed the serum samples for presence of oligomeric abeta using our sandwich ELISA as described in the accompanying file. We analyzed the samples using two different anti-oligomeric abeta nanobodies, A4 and E1. The data shown represents the average oligomeric abeta content in both the A4 and E1 samples (Fig. 28). The samples are presented chronologically and grouped by patient with the diagnostic state shown below each sample. The two control samples are presented on the left and the four AD samples on the right. Three of the four AD samples show a very strong peak in oligomeric abeta levels in the serum samples taken when the patients were cognitively normal, and 2-3 years before the patients converted to MCI and well before conversion to AD. One control sample also showed a strong peak in oligomeric abeta content. We expect that this control person will convert to MCI in the next couple years if the same pattern holds as observed in the AD samples.

### EXAMPLE 4

### 1. Background

We have developed a phage capture ELISA system to distinguish between different neurodegenerative diseases (Williams S, Schulz P, Sierks MR. A Sensitive phage-based capture ELISA for sub-femtomolar detection of protein variants directly from biological samples. Biotechnol. Prog. 2014 Sept. 9). Our system measures the oligomeric content of alpha-synuclein and abeta which are believed to play a role in the progression of the neurodegenerative diseases, Parkinson's (PD) and Alzheimer's (AD), respectively. This system can be also applied to the detection of any other protein variant including aggregated, misfolded, alternatively processed forms. We have currently targeted variants of Tau or TDP-43 (thought to play a role in Amyotrophic Lateral Sclerosis and Frontoemporal dementia). Previously isolated single chain variable fragments (scFvs) that were characterized for their ability to recognize oligomeric forms of alpha-synuclein or abeta were used as the capture antibodies. Targets of interest present in brain tissue, sera and CSF (cerebrospinal fluid) were analyzed in hopes that detection and specificity would be possible, especially in serum, since this represents the most promising source for detection of biomarkers for neurodegenerative diseases. We expect that such sensitivity and detection in serum will provide a very useful diagnostic tool for laboratories to differential between multiple neurological disorders.

### 2. Material and Methods

### Human Samples

Human brain tissue, sera and CSF samples from individuals diagnosed with Parkinson's or Alzheimer's, as well as controls (ND) were acquired from Dr. Thomas Beach at Sun Health Research Institute. Parkinson cases were classified based on the unified staging system for Lewy body disorders (Beach TG, et al. Unified staging system for Lewy body disorders: correlation with nigrostriatal degeneration, cognitive impairment and motor dysfunction. Acta Neuropathol. 2009;117:613-634). The level of alpha-synuclein phosphorylated at serine 129 (p129-syn) in the temporal lobe of the neocortex was evaluated to organize the Parkinson's cases into three groups based on increasing quantities. Braak staging was utilized to assemble the AD cases into two groups; one comprising stages III-IV and the other V-VI. The controls possessed a Braak score of I-II and had zero to moderate plaques.

Nine PDs, six ADs and five control brain tissue cases were available for testing. Regarding sera sample size, eight of the nine PDs, six ADs and four of the five controls matching the tested brain tissue samples numbers were accessible. Similarly, CSF from six of the nine PDs, the six ADs and four of the five controls were on hand for ELISA analysis.

Ante-mortem plasma samples were obtained from Dr. Terrone L. Rosenberry from Mayo Clinic College of Medicine. These samples were longitudinal cases. There were two controls and four AD samples. Controls #1 and #2 had five time points, ADs #1, #2 and #3 has 6 time points and AD #4 had 7 time points.

### Brain Tissue Homogenization

Employing our previously published brain tissue homogenization protocol, the middle temporal gyrus (MTG) was prepared for immunoassay assessment (Williams S, Schulz P, Sierks MR. A Sensitive phage-based capture ELISA for sub-femtomolar detection of protein variants directly from biological samples. Biotechnol. Prog. 2014 Sept. 9). Each sample was chopped and resuspended in homogenization buffer containing 50 mM Tris and 5mM Ethylenediaminetetraacetic acid (EDTA) at pH 7.0. Sonication was then completed at 50% amplitude with 10 seconds pulse on and 15 seconds pulse off for a total of five minutes. After centrifugation at 13,000 rpm for 20 minutes, the supernatants were stored at -80°C for future use.

### scFv Production

The scFvs utilized in this study were produced using a previously published methodology (Williams S, Schulz P, Sierks MR. A Sensitive phage-based capture ELISA for sub-femtomolar detection of protein variants directly from biological samples. Biotechnol. Prog. 2014 Sept. 9). Briefly, HB2151 cells containing the scFv plasmids were cultured overnight in 2xYT, glucose and ampicillin at 37°C with shaking. 10 ml of this overnight culture was added to 1 liter of fresh media and incubated at 37°C with shaking until OD600 was 0.8. After the addition of Isopropyl β-D-1-thiogalactopyranoside (IPTG) the flask was transferred to 30°C. The following day, the supernatant was concentrated using a tangential flow filter (10 kDa filter (Millipore)). The purified scFv was then isolated by means of Fast Protein Liquid Chromatography (FPLC) via a protein A-Sepharose column (GE healthcare, NJ). Following dialysis, the scFvs were stored at - 20°C. SDS-page gel and Western blot analysis was used to confirm purity and presence of the antibodies. Antibody concentrations were calculated using Bicinchoninic Acid (BCA) assay (Pierce, USA).

### Phage Production

Phage particles that were biotinylated and utilized as the detection antibody in the phage capture ELISA system were produced in a similar manner as previously described (Williams S, Schulz P, Sierks MR. A Sensitive phage-based capture ELISA for sub-femtomolar detection of protein variants directly from biological samples. Biotechnol. Prog. 2014 Sept. 9) (MRC Laboratory of Molecular Biology and the MRC Centre for Protein Engineering (Cambridge, UK) - http://www.lifesciences.sourcebioscience.com/media/143421/tomlinsonij.pdf). The scFv displayed on the phage particle have been previously demonstrated to possess reactivity with multiple forms of the target protein (for example reactivity with both monomeric and oligomeric forms of alpha-synuclein). To begin, an overnight culture of the TG1 cells containing our scFv plasmid of interest was diluted 1/100 and cultured in 2xYT containing 100 µg/ml ampicillin and 1% glucose until OD600 was between 0.4 and 0.6. After a 30 minute incubation with 2X1011 of KM13 helper phage or hyperphage (Progen, Germany), the culture was spun and resuspended in 2xYT containing 100 µg/ml ampicillin, 50 µg/ml kanamycin and 0.1% glucose. The cells were cultured at 30°C overnight and the following day centrifuged for 30 minutes at 3000xg. The supernatant was combined with PEG/NaCl and incubated for 1 hour on ice. The solution was again centrifuged at 3000xg for 30 minutes and the pellet saved. The pellet was resuspended in PBS and incubated on ice for 1 hour. Following this incubation step the solution was centrifuged at 11,600xg for 10 minutes and the supernatant stored at -80°C.

The concentration of the phage was estimated using the BCA assay. Once produced, the phages were biotinylated using the EZ-Link Pentylamine-Biotinylation kit (Thermo Scientific, USA). The details of the biotinylation protocol have been published (Williams S, Schulz P, Sierks MR. A Sensitive phage-based capture ELISA for sub-femtomolar detection of protein variants directly from biological samples. Biotechnol. Prog. 2014 Sept. 9).

### Phage Capture ELISA

A comprehensive account of the development of our phage capture ELISA system has been published (Williams S, Schulz P, Sierks MR. A Sensitive phage-based capture ELISA for sub-femtomolar detection of protein variants directly from biological samples. Biotechnol. Prog. 2014 Sept. 9). To begin, the scFvs reactive with oligomeric forms of our proteins of interest were bound to the wells of a high binding ELISA plate (Costar) for 30-60 minutes at 37°. In the case of alpha-synuclein it was 10H and D5 and for abeta it was A4, C6T and E1. After three washes with 0.1% phosphate-buffered saline with tween-20 (PBST), the remaining unbound sites were blocked with 2% milk. Following three washes, the antigen was added. Brain tissue was diluted to 100 µg/ml and for sera and CSF we utilized a 1/100 dilution. The plates were again washed three times and incubated with 200 ng/ml of 40 mmol carboxyl biotinylated phages displaying an scFv that is reactive with most forms of the target protein. The plates were then washed four times and incubated with a 1/1000 dilution of avidin-HRP. After four more washes the binding intensities were detected using the SuperSignal ELISA Femto Maximum Sensitivity Substrate (Thermo Scientific, USA) kit. The signals intensities were quantified using a Wallac Victor2 microplate reader following a 1 and 20 minute development period.

### Statistical Analysis

The raw ELISA data for each sample was divided by the PBS control to produce a signal ratio. The average signal ratio and the standard deviation for the controls were calculated and totaled. Each sample signal ratio was divided by the mean plus one standard deviation (SD) of the controls and any values above 1 were considered positive. All data was evaluated for significance using one-way ANOVA and LSD Post-Hoc analyses made available through the IBM SPSS Statistics 22 data analysis program. The graphs were also generated via this program and table 3 was created using Microsoft Excel 2010.

In Table 3, the cumulative oligomeric alpha-synuclein (10H and D5) and oligomeric abeta (A4 and C6T) were presented. Positive reaction were indicated using the "+" signal and were relative to the control group. The cut-offs were as follows: "+" was more than the mean plus 1 SD, "++" was more than the mean plus 2 SD, "+++" was more than the mean plus 4 SD, "++++" was more than the mean plus 6 SD and "+++++" was more than the mean plus 8 SD.

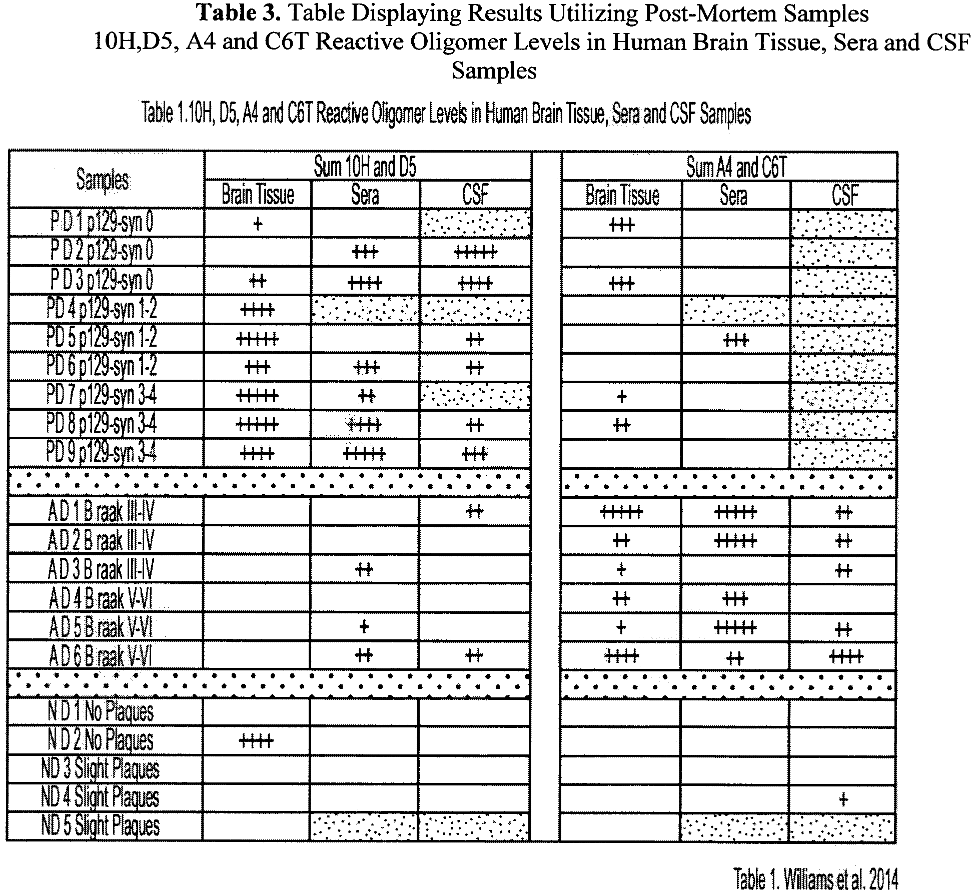

Each star represents a signal that is at least 2 standard deviations stronger than the control signal. The 10H and D5 scFvs that react with a-syn aggregates associated with PD clearly readily pick up the PD samples, with lower reactivity with some of the AD samples. The A4 and C6T scFvs that react with abeta aggregates associated with AD clearly pick up the AD samples with some reactivity with PD samples. None of the scFvs react with ND samples except for one brain tissue sample which is likely an early stage presymptomatic PD patient. It should be noted that a significant number of patients have mixed pathology, for example around 1/3 of AD patients will also suffer from PD. The data shown here suggests that we can detect which patients suffer from multiple diseases.

### 3. Results

### Post-Mortem Human Samples

### Human Brain Tissue Samples

### 10H and D5

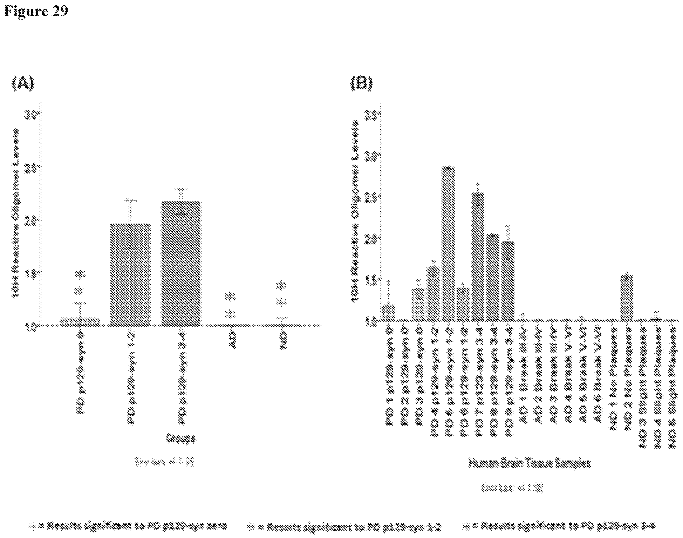

Classification of PD samples based on increasing p129-syn levels in the neocortex revealed a corresponding increase in 10H reactive oligomers levels. The intensities of 10H reactive oligomers for the PD groups with p129-syn 1-2 and p129-syn 3-4 categorization were both significantly different from the PD cases with p129-syn 0, AD and ND groups (Figure 29A). Focusing on the individual samples for each group it can be observed that our 10H ELISA system was selective at isolating 8 out of 9 PD cases and one of the NDs (Figure 29B).

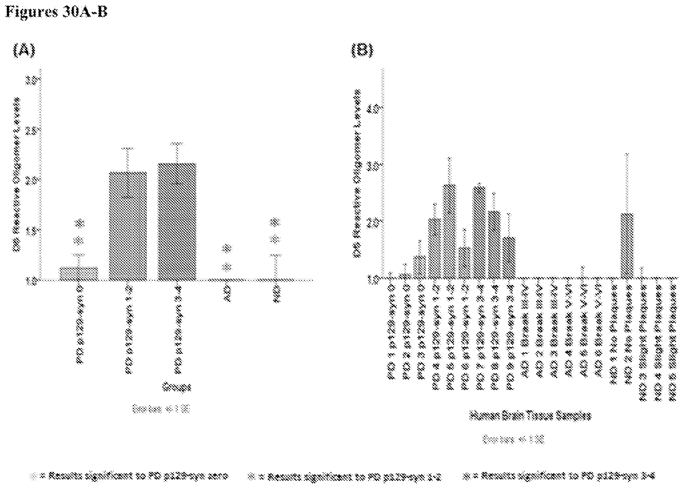

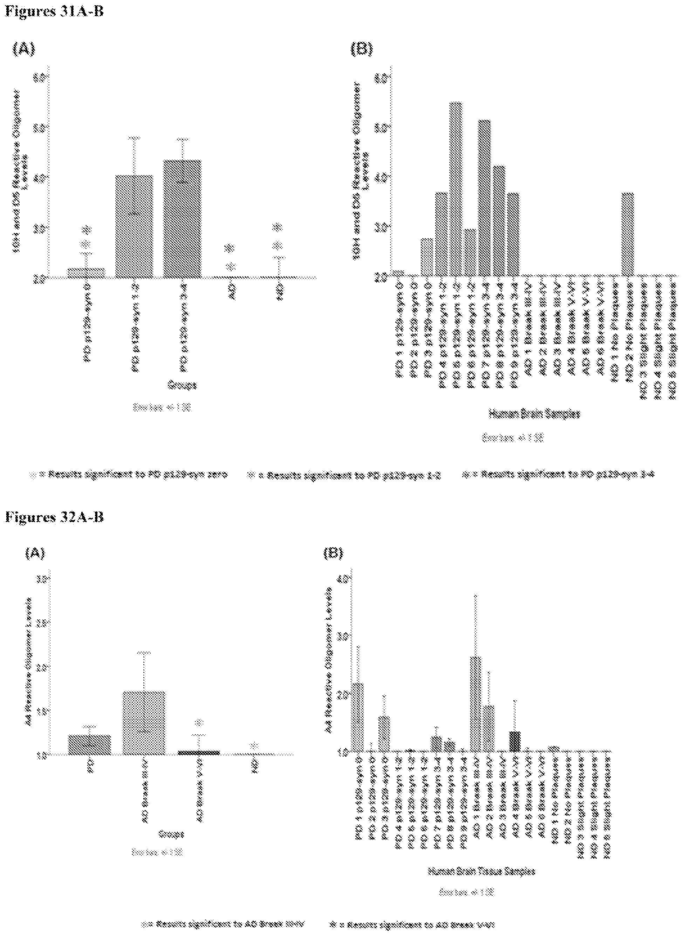

The was also a significant difference in D5 reactive oligomer levels for the PD p129-syn 1-2 and PD p129-syn 3-4 groups compared to the PD p129-syn 0, AD and NDs (Figure 30A). D5 detected 8 out of 9 PD samples and the same ND recognized by 10H (figure 30B). Interestingly, 10H and D5 both selected 8 out of 9 PDs, however, the one PD not detected by 10H was recognized by D5 and vice versa. Therefore, between both scFvs we selected all 9 PD cases. As expected, cumulative 10H and D5 reactive oligomer levels further highlighted the significant difference between the PD p129-syn 1-2 and PD p129-syn 3-4 and the other groups (Figure 31A). Collectively, 10H and D5 also selected 8 out of 9 PDs and the one ND case (Figure 31B).

### A4 and C6T

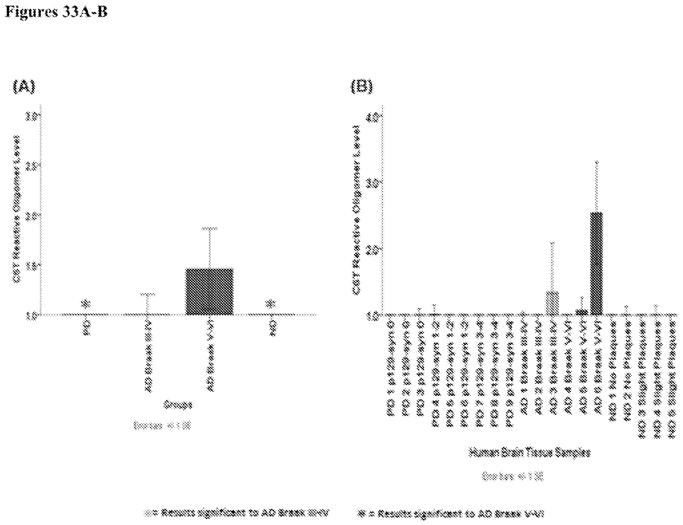

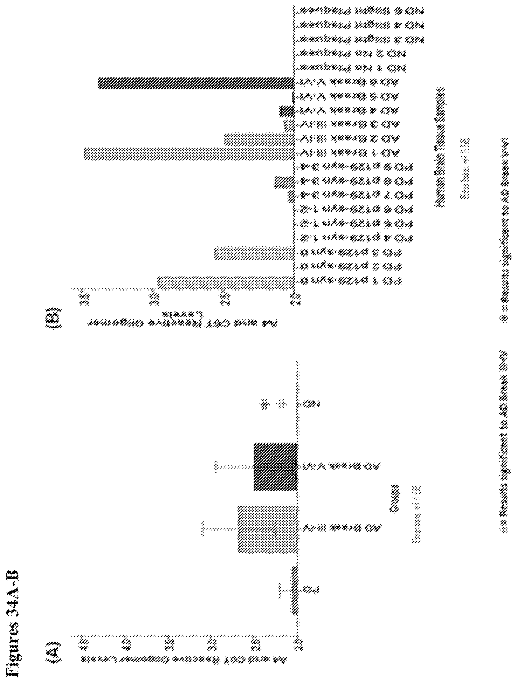

As mentioned, A4 and C6T are antibodies selective for abeta oligomers. A4 detected significantly higher levels of oligomers in the AD group exhibiting Braak stage III-IV compared to the ADs with Braak stage V-VI and NDs (Figure 32A). A4 identified 3 out of 6 AD cases, however, some of the PD cases did yield reactivity with A4 (Figure 32B). Interestingly, most of the PD cases that were selected either displayed microscopic changes of Alzheimer's disease or had a history of dementia in their pathology report. C6T had an overall preference for AD cases with Braak stage V-VI, which was significantly different from the PD and ND groups (Figure 33A). C6T recognized 3 out 6 AD cases, which amazingly were the three AD samples not recognized by A4 (Figure 33B). Cumulative A4 and C6T reactive oligomer levels were significantly different for the AD Braak stage III-IV and AD Braak stage V-VI compared to the controls (Figure 34A). All six AD cases were selected and once again some of the PD cases (Figure 34B).

### Human Sera Samples

### 10H and D5

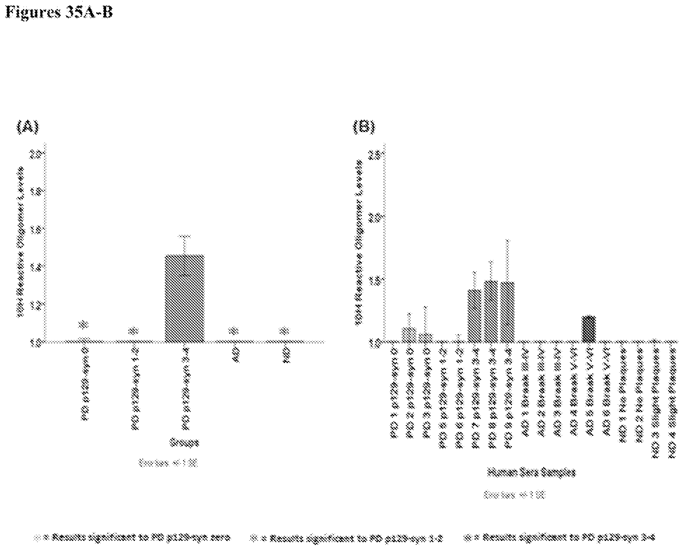

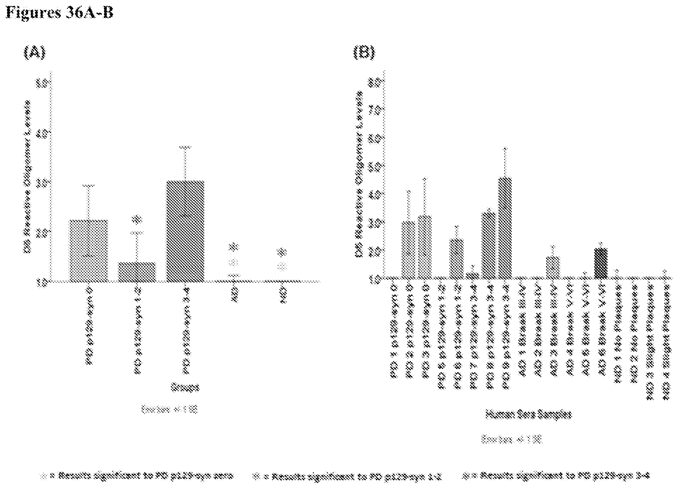

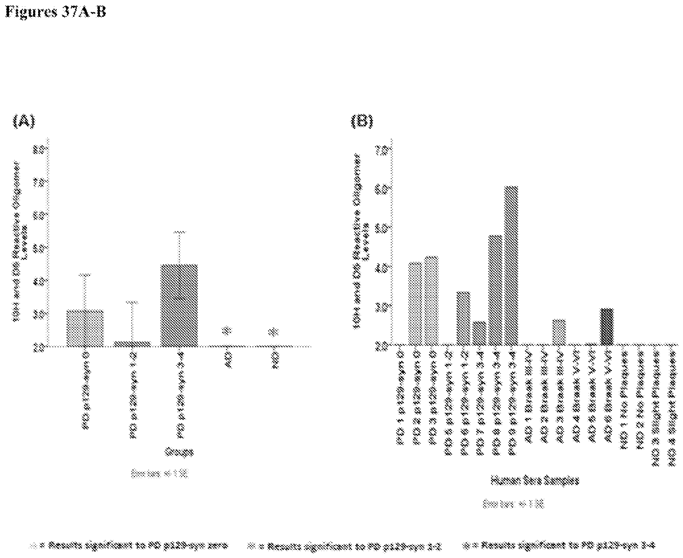

In the sera of these post-mortem samples, our ELISA system was capable of detecting 10H and D5 reactive oligomers. Overall, 10H displayed significantly greater reactivity with the PD p-129 syn 3-4 group compared to the others (Figure 35A). 10H selected two PDs with p129-syn 0 and all three PD cases with p129-syn 3-4 (Figure 35B). One AD case was reactive with 10H. D5 on the other hand showed some level of reactivity with all the PD groups (Figure 36A). The PDs with p129-syn 0 were significantly different from the AD and ND cases and the PDs with p-129 syn 3-4 were significantly different from the PD p129-syn 1-2, AD and ND groups. D5 selected 6 out of 8 PD cases and two AD samples (Figure 36B). Cumulatively, 10H and D5 reacted with all PD groups and selected 6 out of 8 PD cases and three AD cases (Figure 37A and 37B). In table 3, which summarizes the cumulative scFv reactivity with the different biological mediums, more "+" signs indicated higher level of reactivity based on the mean plus various standard deviations from the controls. Even though the total 10H and D5 levels displayed some reactivity with the AD samples, the PD cases selected had greater number of "+" showing greater preference for PDs with these scFvs.

### A4 and C6T

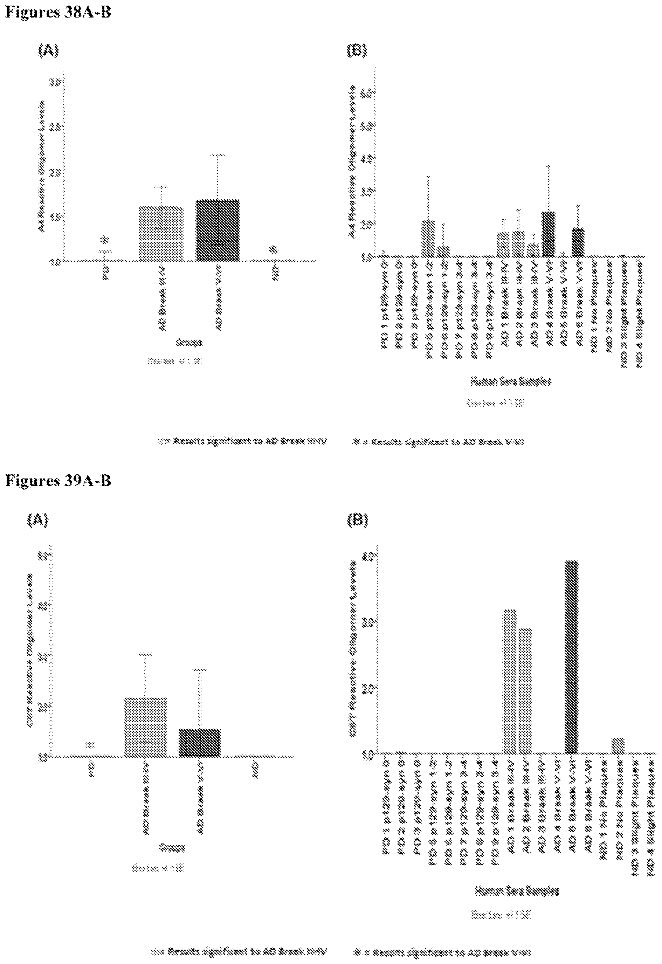

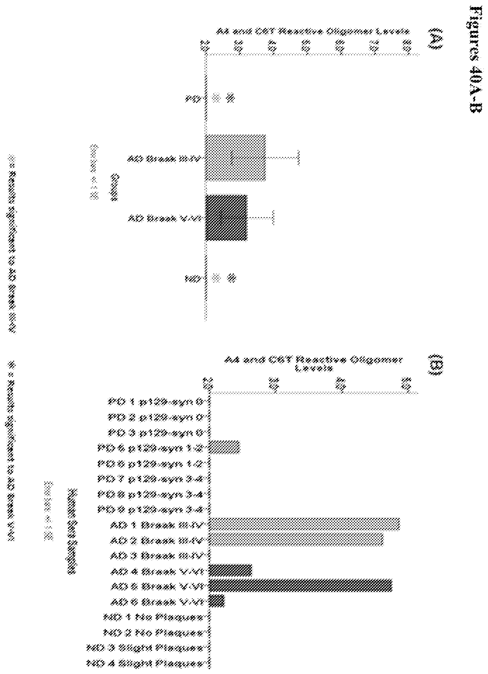

Utilizing A4 scFv, both AD groups displayed reactivity and ADs with Braak stage V-VI had significantly greater oligomer content compared to the PDs and NDs (Figure 38A). A4 selected 5 out of 6 AD cases and three of the PDs (which again were the samples with microscopic changes of Alzheimer's disease or had a history of dementia in their pathology report) (Figure 38B). Both AD groups also displayed some overall reactivity with C6T, but the ADs with Braak stage III-IV were significantly different from the PDs (Figure 39A). C6T selected 3 out of 6 AD cases and a different PD sample (Figure 39B). Between A4 and C6T all AD cases were selected. Cumulatively, A4 and C6T reacted with both AD groups and both groups were significantly different from the PD and ND cases (Figure 40A). Five out of 6 AD cases were selected when totaling A4 and C6T along with only one PD case (Figure 40B). This may indicate that utilizing total oligomer content may be a more stringent method to ensure better selectivity of cases. Table 3, further demonstrated that collectively A4 and C6T displayed more selectivity for AD cases since more "+" signs were exhibited for these samples.

### Human CSF Samples

### 10H and D5

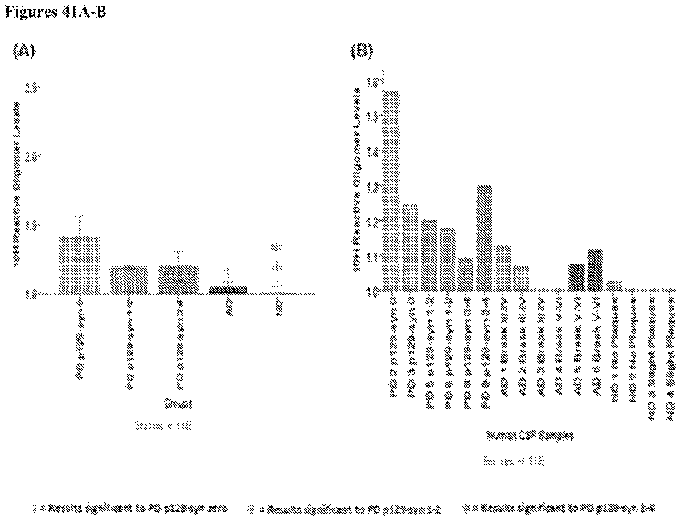

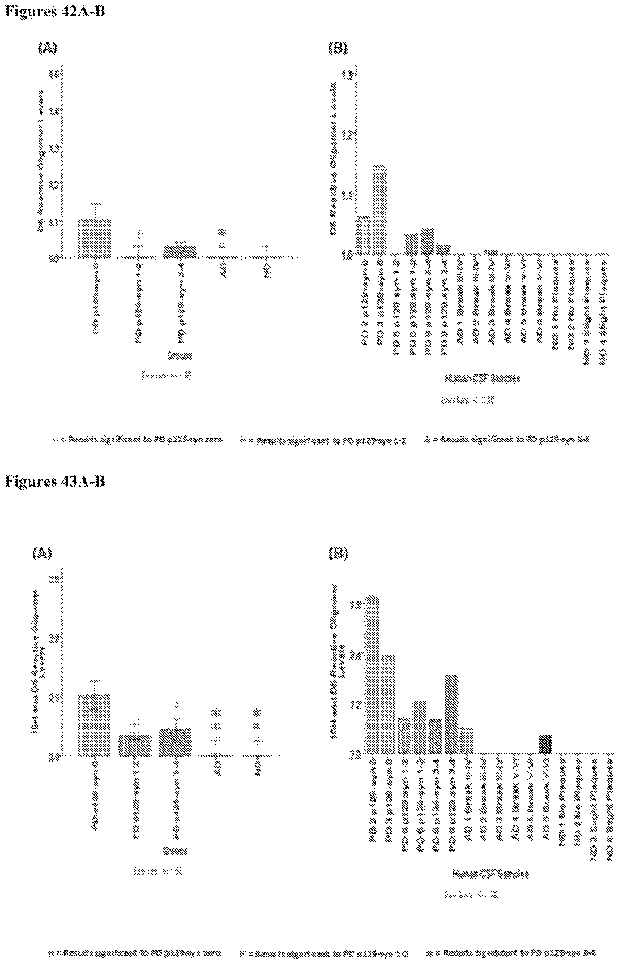

In the CSF, 10H reacted significantly more with the PDs with p129-syn 0 compared to the AD and ND groups (Figure 41A). The PD p129-syn 1-2 and PD p129-syn 3-4 groups were also significantly different from the NDs. 10H selected all 6 PD cases, but also some of the ADs and one ND (Figure 41B). With D5, PD p129-syn 0 also had significantly higher signals compared to all groups except PD p129-syn 3-4, which was also significantly different from the ADs (Figure 42A). D5 on the other hand selected 5 out of 6 PDs and only one AD (Figure 42B). This AD was the same sample D5 selected in the capture ELISA using sera (Figure 36B). Cumulatively, 10H and D5 displayed reactivity with all PD groups and they were all significantly different from the ADs and NDs (Figure 43A). PD p129-syn 0 was also significantly different from the PD p-129 syn 1-2 and PD p129-syn 3-4 groups. All 6 PD cases were selected along with only two AD cases (Figure 43B). Table 3 again demonstrated that cumulatively 10H and D5 displayed a preference for PD CSF samples.

### A4 and C6T

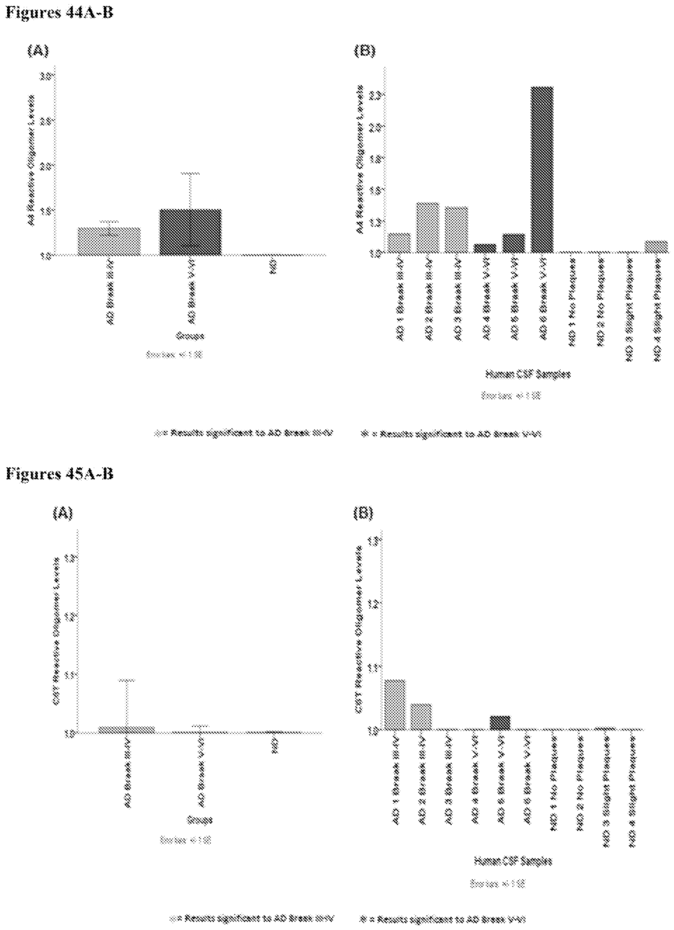

Due to insufficient PD CSF samples, we were only able to test A4 and C6T with the AD and ND cases. A4 was reactive with both AD groups and selected all 6 AD cases and only one control (Figures 44A and B). Overall C6T was reactive with the AD group exhibiting Braak stage III-IV and selected 3 out of 6 AD cases and another ND (Figures 45A and B). Cumulatively, A4 and C6T reacted with both AD groups and selected 5 out of 6 AD cases and one ND (Figures 46A and B). Table 3 indicated that the level of reactivity with the control sample was lower than with the AD cases (only one "+" sign).

### Ante-Mortem Human Samples

### Human Plasma Samples

### A4 and E1

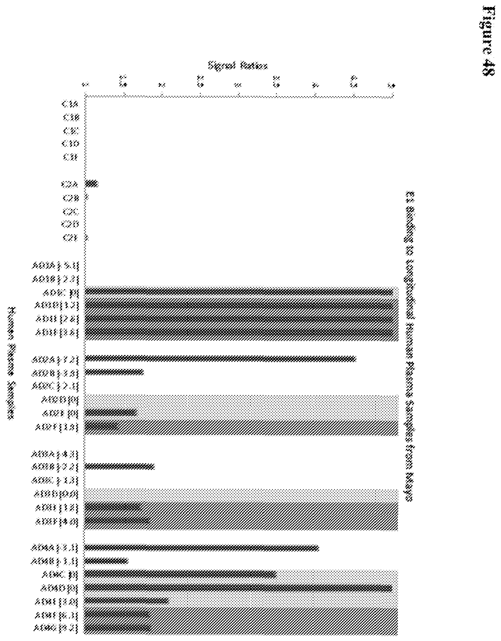

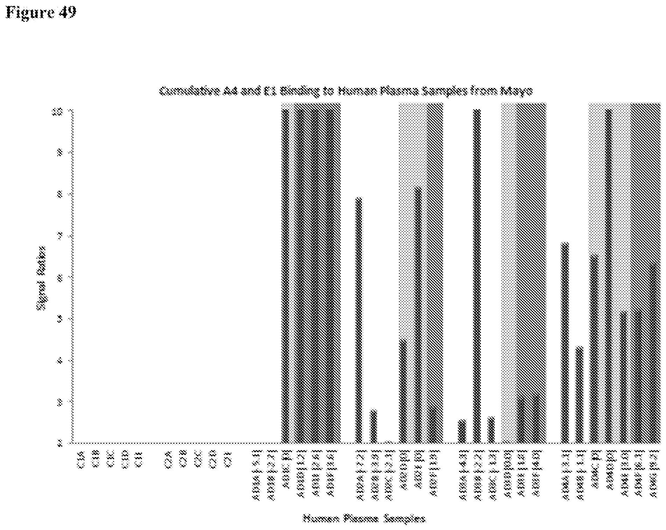

The A4 and E1 oligomeric content of longitudinal human plasma samples were analyzed. E1 should also be reactive with abeta oligomers. There were two controls and four AD cases. For each we tested plasma samples from multiple time points and in the AD cases we tested pre-MCI (mild cognitive impairment), during MCI (background in light purple) and after MCI (background in dark purple). Both A4 and E1 reacted greater with the four AD cases (Figures 47 and 48) compared to the controls. Cumulatively, A4 and E1 selected every time point for all the AD cases except AD #1 (Figure 49). What is remarkable though is the detection of oligomer content pre-MCI and during MCI and almost no significant reactivity with the controls.

### 4. Summary

Overall, our phage capture ELISA system was beneficial in the detection of the oligomeric content in different neurodegenerative diseases. Using the post-mortem human brain tissue, sera and CSF samples, 10H and D5 displayed more selectivity for PD samples as expected and A4 and C6T displayed more selectivity for AD samples. Table 3 summarizes the level of preference these scFvs permitted. In ante-mortem samples, A4 and E1 also react more with the AD samples when compared to the controls and even more importantly pre-MCI.

The detection in sera and CSF is significant because this allows for the use of our antibodies and the ELISA system as diagnostic tools. Because serum and plasma are easily attainable for testing compared to CSF, this helps to make diagnosis more painless in the future. While in the foregoing specification this invention has been described in relation to certain embodiments thereof, and many details have been set forth for purposes of illustration, it will be apparent to those skilled in the art that the invention is susceptible to additional embodiments and that certain of the details described herein may be varied considerably without departing from the basic principles of the invention.

The use of the terms "a" and "an" and "the" and similar referents in the context of describing the invention are to be construed to cover both the singular and the plural, unless otherwise indicated herein or clearly contradicted by context. The terms "comprising," "having," "including," and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Embodiments of this invention are described herein. Variations of those embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of determining a quantity of a target oligomer in a biological fluid sample from a patient to determine an increased likelihood of having or developing a neurodegenerative disease, comprising;
(a) contacting the sample to a solid support, wherein the solid support is coated with a capture agent specific for the target oligomer and is blocked with a blocking agent to form a bound sample;
(b) incubating the bound sample;
(c) contacting the bound sample with labelled phage specific for the target oligomer to form a labeled bound sample;
(d) contacting the labeled bound sample with labeled detection agent to form a visualizable sample; and
(e) comparing the quantity of the visualizable sample and to a control to determine the quantity of the target oligomer.

2. A method of distinguishing between different neurodegenerative diseases in a biological fluid sample from a patient, comprising:
(a) contacting the sample to a solid support, wherein the solid support comprises two or more locations, wherein each location is coated with a different capture agent specific for a target oligomer, and wherein the solid support is blocked with a blocking agent to form a bound sample;
(b) incubating the bound sample;
(c) contacting the bound sample with two or more labelled phages, wherein each labelled phage is specific for a single target oligomer to form labeled bound samples;
(d) contacting the labeled bound samples with labeled detection agent to form a visualizable samples; and
(e) comparing the quantity of the visualizable samples to quantities of controls to determine the quantity of each target oligomer;
wherein an increase in toxic oligomeric Aβ is indicative of the patient having early AD,
wherein an increase in oligomeric Aβ and tau aggregates is indicative of later stage AD,
wherein an increase in oligomeric tau but not oligomeric Aβ is indicative a tauopathy;
wherein an increase in oligomeric a-syn is indicative of Parkinson's disease; and wherein an increase oligomeric Aβ and a-syn is indicative of Dementia with Lewy Bodies (DLB).

3. The method of any one of claims 1 or 2, wherein the biological fluid is blood, cerebral spinal fluid (CSF), saliva, tears, urine, or any bodily fluid.

4. The method of any one of claims 1 to 3, wherein the capture agent is an antibody.

5. The method of claim 4, wherein the antibody is a single chain variable fragment (scFv).

6. The method of any one of claims 1 to 5, wherein the capture agent is scFv-A4, scFv-C6T, scFv-10H, or scFv-E1.

7. The method of any one of claims 1 to 6, wherein the blocking agent is milk, preferably a concentration of 0.5% to 10% milk.

8. The method of any one of claims 1 to 7, wherein the bound sample in step (b) is incubated for at least one hour.

9. The method of claim 8, wherein the bound sample in step (b) is incubated for about two hours.

10. The method of any one of claims 1 to 9, wherein the bound sample in step (b) is incubated at 30°C to 40°C.

11. The method of any one of claims 1 to 10, wherein the phage is at a concentration range of 10⁻³ to 10⁻⁵.

12. The method of any one of claims 1 to 10, wherein the phage is at a concentration of about 10⁻⁴.

13. The method of any one of claims 1 to 12, wherein the phage specific for the target oligomer is SRP-phage.

14. The method of any one of claims 1 to 13, wherein the quantity detected is in a fentomolar ratio.

## Patentansprüche

1. Verfahren zum Bestimmen einer Menge eines Zieloligomeren in einer biologischen, fluiden Probe von einem Patienten, um eine erhöhte Wahrscheinlichkeit zum Aufweisen oder Entwickeln einer neurodegenerativen Erkrankung zu ermitteln, umfassend;
(a) Kontaktieren der Probe mit einem festen Träger, wobei der feste Träger mit einem Bindungsmittel beschichtet ist, welches für das Zieloligomer spezifisch ist, und mit einem Blockierungsmittel blockiert wird, um eine gebundene Probe zu bilden;
(b) Inkubieren der gebundenen Probe;
(c) Kontaktieren der gebundenen Probe mit markierten Phagen, die für das Zieloligomer spezifisch sind, um eine markierte gebundene Probe zu bilden;
(d) Kontaktieren der markierten gebundenen Probe mit einem markierten Nachweismittel, um eine visualisierbare Probe zu bilden; und
(e) Vergleichen der Menge der visualisierbaren Probe mit einer Kontrollmenge, um die Menge des Zieloligomeren zu bestimmen.

2. Verfahren zum Unterscheiden zwischen verschiedenen neurodegenerativen Erkrankungen in einer biologischen, fluiden Probe von einem Patienten, umfassend:
(a) Kontaktieren der Probe mit einem festen Träger, wobei der feste Träger zwei oder mehr Stellen aufweist, wobei jede Stelle mit einem unterschiedlichen Bindungsmittel beschichtet ist, das für ein Zieloligomer spezifisch ist, und wobei der feste Träger mit einem Blockierungsmittel blockiert wird, um eine gebundene Probe zu bilden;
(b) Inkubieren der gebundenen Probe;
(c) Kontaktieren der gebundenen Probe mit zwei oder mehr markierten Phagen, wobei jede markierte Phage für ein einzelnes Zieloligomer spezifisch ist, um markierte gebundene Proben zu bilden;
(d) Kontaktieren der markierten gebundenen Proben mit einem markierten Nachweismittel, um visualisierbare Proben zu bilden; und
(e) Vergleichen der Menge der visualisierbaren Proben mit Kontrollmengen, um die Menge von jedem Zieloligomer zu bestimmen;
wobei eine Zunahme an toxischem, oligomerem Aβ hinweisgebend ist auf einen Patienten mit AD im Frühstadium,
wobei eine Zunahme an oligomerem Aβ und Tau-Aggregaten hinweisgebend ist auf AD im Spätstadium,
wobei eine Zunahme an oligomerem Tau, aber nicht oligomerem Aβ, hinweisgebend ist auf eine Tauopathie;
wobei eine Zunahme an oligomerem a-syn hinweisgebend ist auf eine Parkinson-Krankheit; und
wobei eine Zunahme an oligomerem Aβ und a-syn hinweisgebend ist auf eine Demenz mit Lewy-Körpern (DLB).

3. Verfahren nach einem der Ansprüche 1 oder 2, wobei die biologische Flüssigkeit Blut, Rückenmarksflüssigkeit (CSF), Speichel, Tränen, Urin, oder eine andere Körperflüssigkeit ist.

4. Verfahren nach einem der Ansprüche 1 bis 3, wobei das Bindungsmittel ein Antikörper ist.

5. Verfahren nach Anspruch 4, wobei der Antikörper ein einkettiges, variables Fragment (scFv) ist.

6. Verfahren nach einem Ansprüche 1 bis 5, wobei das Bindungsmittel scFv-A4, scFv-C6T, scFv-10H oder scFv-E1 ist.

7. Verfahren nach einem der Ansprüche 1 bis 6, wobei das Blockierungsmittel Milch ist, bevorzugt mit einer Konzentration von 0,5% bis 10% an Milch.

8. Verfahren nach einem der Ansprüche 1 bis 7, wobei die gebundene Probe in Schritt (b) für zumindest eine Stunde inkubiert wird.

9. Verfahren nach Anspruch 8, wobei die gebundene Probe in Schritt (b) für etwa zwei Stunden inkubiert wird.

10. Verfahren nach einem der Ansprüche 1 bis 9, wobei die gebundene Probe in Schritt (b) bei 30°C bis 40°C inkubiert wird.

11. Verfahren nach einem der Ansprüche 1 bis 10, wobei der Phage in einer Konzentration von 10⁻³ bis 10⁻⁵ vorliegt.

12. Verfahren nach einem der Ansprüche 1 bis 10, wobei der Phage in einer Konzentration von etwa 10⁻⁴ vorliegt.

13. Verfahren nach einem der Ansprüche 1 bis 12, wobei der Phage, der spezifisch für das Zieloligomer ist, ein SRP-Phage ist.

14. Verfahren nach einem der Ansprüche 1 bis 13, wobei die bestimmte Menge in einem femtomolaren Verhältnis vorliegt.

## Revendications

1. Procédé de détermination de la quantité d'un oligomère cible dans un échantillon de fluide biologique d'un patient pour déterminer un risque accru d'avoir ou de développer une maladie neurodégénérative, comprenant :
(a) la mise en contact de l'échantillon avec un support solide, où le support solide est recouvert d'un agent de capture spécifique de l'oligomère cible et est bloqué à l'aide d'un agent bloquant pour former un échantillon lié ;
(b) l'incubation de l'échantillon lié ;
(c) la mise en contact de l'échantillon lié avec un phage marqué, spécifique de l'oligomère cible pour former un échantillon lié marqué ;
(d) la mise en contact de l'échantillon lié marqué avec un agent de détection marqué pour former un échantillon visualisable ; et
(e) la comparaison de la quantité d'échantillon visualisable avec un témoin pour déterminer la quantité de l'oligomère cible.

2. Procédé permettant de faire la distinction entre différentes maladies neurodégénératives dans un échantillon de fluide biologique d'un patient, comprenant :
(a) la mise en contact de l'échantillon avec un support solide, où le support solide comporte deux emplacements ou plus, chaque emplacement étant recouvert d'un agent de capture différent, spécifique d'un oligomère cible, et où le support solide est bloqué à l'aide d'un agent bloquant pour former un échantillon lié ;
(b) l'incubation de l'échantillon lié ;
(c) la mise en contact de l'échantillon lié avec deux phages marqués ou plus, chaque phage marqué étant spécifique d'un seul oligomère cible pour former des échantillons liés marqués ;
(d) la mise en contact des échantillons liés marqués avec un agent de détection marqué pour former des échantillons visualisables ; et
(e) la comparaison de la quantité des échantillons visualisables avec celles des témoins pour déterminer la quantité de chaque oligomère cible ;
dans lequel une augmentation de l'Aβ oligomérique toxique indique que le patient est aux premiers stades de la maladie d'Alzheimer (MA),
dans lequel une augmentation de l'Aβ oligomérique et des agrégats de protéine tau indique une MA au stade ultérieur,
dans lequel une augmentation de tau oligomérique mais pas d'Aβ oligomérique indique une tauopathie ;
dans lequel une augmentation de l'α-syn oligomérique indique une maladie de Parkinson ; et
dans lequel une augmentation des Aβ et α-syn oligomériques indique une démence à corps de Lewy (DLB).

3. Procédé selon l'une quelconque des revendications 1 et 2, dans lequel le fluide biologique et du sang, du liquide céphalo-rachidien (LCR), de la salive, des larmes, de l'urine ou n'importe quel fluide corporel.

4. Procédé selon l'une quelconque des revendications 1 à 3, dans lequel l'agent de capture est un anticorps.

5. Procédé selon la revendication 4, dans lequel l'anticorps est un fragment variable à chaîne unique (scFv).

6. Procédé selon l'une quelconque des revendications 1 à 5, dans lequel l'agent de capture est scFv-A4, scFv-C6T, scFv-10H ou scFv-E1.

7. Procédé selon l'une quelconque des revendications 1 à 6, dans lequel l'agent bloquant est du lait, de préférence une concentration de lait à 0,5% à 10%.

8. Procédé selon l'une quelconque des revendications 1 à 7, dans lequel l'échantillon lié de l'étape (b) est mis à incuber pendant au moins une heure.

9. Procédé selon la revendication 8, dans lequel l'échantillon lié de l'étape (b) est mis à incuber pendant environ deux heures.

10. Procédé selon l'une quelconque des revendications 1 à 9, dans lequel l'échantillon lié de l'étape (b) est mis à incuber à une température de 30°C à 40°C.

11. Procédé selon l'une quelconque des revendications 1 à 10, dans lequel le phage est à une concentration comprise dans l'intervalle de 10⁻³ à 10⁻⁵.

12. Procédé selon l'une quelconque des revendications 1 à 10, dans lequel le phage est à une concentration d'environ 10⁻⁴.

13. Procédé selon l'une quelconque des revendications 1 à 12, dans lequel le phage spécifique de l'oligomère cible est un phage à SRP.

14. Procédé selon l'une quelconque des revendications 1 à 13, dans lequel la quantité détectée est dans un rapport femtomolaire.
